# EUROPEAN PATENT APPLICATION

(11) **EP 4 541 815 A1**
(43) Date of publication of application: **23.04.2025**
(21) Application number: 23826339.6
(22) Date of filing: 19.06.2023
(51) Int. Cl.: C07K 16/18, C12N 15/12, C12N 15/63, A61K 39/395, A61P 31/12, A61P 1/16

(54) **ANTIBODY AGAINST HEPATITIS B VIRUS, PREPARATION THEREOF AND USE THEREOF**

(30) Priority: 20.06.2022 CN 202210699418
(71) Applicant: Huahui Health Ltd., Beijing 102206 (CN)
(72) Inventor: QI, Yonghe, Beijing 102206 (CN); HAO, Dongxia, Beijing 102206 (CN)
(74) Representative: Rückerl, Florian
(86) International application number: PCT/CN2023/101035
(87) International publication number: WO 2023/246691

(57) **Abstract**

The present invention provides an antibody against the hepatitis B virus. The antibody has enhanced Fc-mediated effector functions, such as ADCC and ADCP activity, and optionally further has a prolonged half-life. Also disclosed are a method and a cell for producing the antibody, and use of the antibody.

## Description

### Technical Field

The present invention belongs to the field of antibody drugs. The present invention provides an antibody variant specifically targeting hepatitis B virus (HBV). The antibody variant has enhanced Fc-mediated effector functions, such as ADCC and/or ADCP activity relative to the parent antibody, and optionally further has a prolonged *in vivo* half-life. The present invention further provides a method and a cell for producing the antibody variant. The present invention further provides a pharmaceutical composition comprising the antibody variant, use of the antibody variant, and combination of the antibody variant with other anti-HBV drugs.

### Background Art

Hepatitis B virus (HBV) infection is the main cause of liver disease. More than 296 million people are chronically infected with HBV worldwide, out of which about 820,000 people die every year from liver cirrhosis or hepatocellular carcinoma caused by hepatitis B virus infection (Hepatitis B (who.int) https://www.who.int/news-room/fact-sheets/detail/hepatitis-b). Although universal vaccination against hepatitis B can effectively reduce new infections, and interferon and nucleos(t)ide anti-hepatitis B drugs can effectively inhibit viral replication, they fail to eliminate the hepatitis B virus completely and achieve the goal of curing chronic hepatitis B. Therefore, long-term administration is needed for majority of patients with chronic hepatitis B, and even patients with sustained virological suppression may still develop hepatocellular carcinoma (Grossi, G., et al. (2017). Hepatitis B virus long-term impact of antiviral therapy nucleot(s)ide analogues (NUCs). Liver Int 37 Suppl 1: 45-51).

A14 antibody is a fully human IgG1 monoclonal antibody targeting the pre-S1 region of hepatitis B virus (HBV), which not only inhibits the entry of hepatitis B virus into hepatocytes, but also has the ability of Fc-mediated immunological effector functions and thus elimination of the hepatitis B virus or infected cell (WO2016188386A1), and is a potential inhibitor for the treatment of chronic hepatitis B (WO2021013135A1).

In the pharmaceutical industry, antibody drugs are usually produced using an animal cell as the production host cell. It is known that the Fc effector functions of IgG1 antibodies, such as antibody-dependent cell-mediated cytotoxicity (ADCC) and complement-dependent cytotoxicity (CDC) are affected by glycosylation. In addition, it is also believed that the structure of sugar chains in glycosylation is determined by glycosyltransferase genes involved in sugar chain synthesis and glycosidases involved in sugar chain hydrolysis in the production host cell. Researchers have proposed that the effector functions of IgG antibodies, particularly those of the IgG1 subclass, can be altered by changing glycosylation modifications.

The inventor of US8067232B2 found that antibodies of fucose-free sugar chains showed higher ADCC activity, and the lower the content of 1,6-fucose sugar chain, the stronger the ADCC activity thereof. The patent provides mammalian cell, such as CHO cell, in which α-1,6-fucosyltransferase is inactivated by homologous recombination, and demonstrates that antibodies produced using such cell have enhanced ADCC activity.

US7662925 discloses various Fc mutations that may alter the binding affinity of IgG1 antibodies to human Fcy receptors.

However, given the complexity of the structure and function of antibody protein moleculars, as well as the diversity and complexity of sugar chain structures, it remains difficult to predict in advance which amino acid sites or types of glycosylation modifications can be altered to adjust the effector function, as well as other properties of a particular antibody to a desired extent. Thus, there is an unmet need for an anti-HBV antibody with improved property and production method thereof.

### Summary of the Invention

The inventors of the present invention have conducted extensive exploration and developed an anti-hepatitis B virus antibody variant with enhanced Fc effector function and prolonged half-life based on the A14 antibody, thereby completing the present invention.

Accordingly, in a first aspect, the present invention relates to an IgG1 antibody variant that specifically binds to a Pre-S1 domain of hepatitis B virus (HBV) and has enhanced affinity with activating Fcγ receptors (FcγRs) and reduced affinity with inhibitory Fcγ receptors (FcγR) compared to the parent antibody, thereby possessing enhanced Fc-mediated effector functions, such as enhanced ADCC and/or ADCP activity.

In some specific embodiments, relative to the parent antibody, the antibody variant comprises one or more amino acid mutations in the heavy chain constant region with respect to the heavy chain constant region of human IgG1 as shown by the amino acids of SEQ ID NO: 31, and optionally, a sugar chain containing 1,6-fucose is reduced or absent in the antibody variant. In some specific embodiments, the amino acid mutations are located in Fc region of the antibody variant, which corresponds to the sequence of amino acids from positions 114 to 330 in SEQ ID NO: 31. In some preferred embodiments, the amino acid mutations are selected from one or more of the following groups: G236A, S239D, I332E, F243L, R292P, Y300L, V305I, P396L, and L235V, in which the amino acid position is determined according to the EU numbering.

Further, the antibody variant comprising an amino acid mutation in the Fc region has a prolonged half-life compared to the unmodified antibody. In some specific embodiments, the antibody variant further comprises one or more amino acid mutations selected from the group consisting of: M252Y, S254T, T256E, M428L, and N434S.

In a second aspect, the present invention provides a production cell for preparing an afucosylated antibody, wherein the cell is a mammalian cell with an inactivated FUT8 gene, such as a 293F cell, a CHOK1 cell, a CHOZN GS-/- cell, or a CHO K1Q cell; and the cell is introduced a sequence encoding the antibody. Preferably, the cell is 293F cell, CHOK1 cell, CHOZN GS-/- cell or CHO K1Q cell with *Fut8* gene knockout. Preferably, the introduced sequence encodes an antibody variant described in the first aspect.

In a third aspect, the present invention provides a method for preparing a production cell with *Fut8* gene knockout, comprising introducing into a mammalian cell (a) a gRNA targeting *Fut8* and a coding sequence of Cas9 nuclease, or (b) a complex of a gRNA targeting *Fut8* and Cas9 nuclease.

In a fourth aspect, the present invention provides an sgRNA, which is used to prepare a cell with *Fut8* gene knockout.

In a fifth aspect, the present invention provides a method for preparing an antibody, comprising culturing the cell of the second aspect in a medium to produce a culture comprising an antibody.

In a sixth aspect, the present invention provides an antibody prepared by the method of the fifth aspect.

In a seventh aspect, the present invention provides an isolated nucleic acid molecule comprising a nucleotide sequence encoding the antibody of the first aspect.

In an eighth aspect, the present invention provides an expression vector comprising the isolated nucleic acid molecule of the seventh aspect.

In a ninth aspect, the present invention provides a host cell comprising the isolated nucleic acid molecule of the seventh aspect or the expression vector of the eighth aspect.

In a tenth aspect, the present invention provides a pharmaceutical composition comprising the antibody variant of the first aspect or the antibody of the sixth aspect.

In an eleventh aspect, the present invention provides the use of the antibody variant of the first aspect or the antibody of the sixth aspect for the preparation of a medicament, the medicament being used in a subject in need thereof for treating hepatitis B or D virus infection, or a disease caused by the infection, such as chronic hepatitis B, or for eliciting an Fc effector function.

In a twelfth aspect, the present invention provides a method for treating hepatitis B or D virus infection or a disease caused by such infections, such as chronic hepatitis B, or for eliciting an ADCC effect in a subject, comprising administering to the subject the antibody variant of the first aspect or the antibody of the sixth aspect.

In a thirteenth aspect, the present invention provides a combination of the antibody variant of the first aspect or the antibody of the sixth aspect with another antiviral drug.

### Brief Description of Drawings

Figures 1A-B are the plasmid maps of pLenti-sgFut8-EGFP (A) and pLenti-OCT1-cas9-IRES-BSD (B), respectively.
Figure 2 is a gel image of a semi-quantitative assay to detect the efficiency of gene editing.
Figure 3 is a curve plot showing the binding ability of FITC-labeled Lens Culinaris Agglutinin (LCA) to 293F wild-type cells and *Fut8* gene-edited cells by FACS analysis.
Figure 4 shows the DNA sequence of sgRNA-targeted *Fut8* region in FCC# cell as analyzed by Sanger sequencing.
Figure 5 is a curve plot showing the binding ability of PE-LCA to CHOK1 wild-type cell, *Fut8* gene-edited cell as analyzed by FACS.
Figure 6 shows the results of cleavage of Fut8 DNA fragments with the sgcgFut8-2 gRNA/Cas9 complex *in vitro.*
Figure 7 shows the results of the efficiency of Fut8 gene editing in CHOZN GS-/- cell using the sgcgFut8-2 gRNA/Cas9 verified by T7E1 digestion.
Figure 8 shows the results of FITC-LCA binding to CHOZN GS-/- Fut8 gene-edited cells as analyzed by FACS.
Figure 9 shows the results of the binding ability of FITC-LCA to CHOZN GS-/- Fut8_KO-8# cell as analyzed by flow cytometry.
Figure 10 shows the results of Fut8 DNA sequence targeted by sgcgFut8-2 in CHOZN GS-/- Fut8_KO-8# cell as analyzed by Sanger sequencing.
Figure 11 shows the results of the efficiency of sgcgFut8-2 gRNA/Cas9 editing of the Fut8 gene in CHO K1Q cell verified by T7E1 digestion.
Figure 12 shows the results of the binding ability of FITC-LCA to wild-type CHO K1Q cell and a afucosylated cell line thereof as analyzed by flow cytometry.
Figures 13A-C show the results of DNA sequence of Fut8 region targeted by sgRNA in afucosylated CHO K1Q cell as analyzed by Sanger sequencing. (A) CHO K1Q-Fut8 KO-1#; (B) CHO K1Q-Fut8 KO-2#; and (C) CHO K1Q-Fut8 KO-3#.
Figure 14 shows ADCC and ADCP activities *in vitro* of individual A14 mutant antibodies.
Figure 15 shows dose-dependent ADCC activity *in vitro* of individual candidate mutant antibodies.
Figure 16 shows ADCP activity *in vitro* of individual A14 mutant antibodies.
Figure 17 shows the results of recombinant expression of human FcyRs protein *in vitro* as analyzed by SDS-PAGE.
Figure 18 shows ADCC and ADCP activities *in vitro* of the mutant antibody of Example 5.
Figure 19 shows the results of expressed and purified A14 WT and afuco-A14 GA LS as analyzed by SDS-PAGE.
Figure 20 shows ADCC and ADCP activities *in vitro* of afuco-A14 GA LS.
Figure 21 shows the results of the affinity of afuco-A14 GA LS to human FcRn as analyzed by Biacore.
Figure 22 shows the results of the binding activity of afuco-A14 GA LS to the target antigen Pre-S1 as analyzed by ELISA.
Figure 23 shows the antiviral activity of afuco-A14 GA LS *in vitro.*
Figure 24 shows the half-life of afuco-A14 GA LS in hFcRn KI mice.

### Detailed Description of the Invention

### Definitions

"Antibody" in the context of the present invention has the meaning known in the art, i.e. an immunoglobulin with a Y-shaped structure produced by the immune system as a response to an exogenous substance such as a pathogen. The antibody of classical structure is a homodimer, with each monomer comprising a heavy chain and a light chain linked by disulfide bonds. The light chain consists of one variable region (V_{L}) and one constant region (C_{L}), while the heavy chain consists of one variable region (V_{H}) and three constant regions (C_{H}1, C_{H}2 and C_{H}3). Each variable region contains three complementarity determining regions (CDRs), which form the antigen-binding site responsible for complementarity with the antigen. The antigen-binding fragment composed of V_{L}, C_{L}, V_{H}, and C_{H}1 is known as "Fab"region. The remaining part, which corresponds to the "trunk" below the Y-shaped structure, is called the "Fc" region and consists of the C_{H}2 and C_{H}3 domains of the heavy chain constant regions of the antibody.

The "Fc region" has various functions, including binding to effector cell via the Fc receptors (FcRs) on effector cell such as macrophage or natural killer cell thereby activating the immune effector pathway. In addition, the Fc region comprises glycosylation sites, and the sugar chains attached to these glycosylation sites can also influence the Fc effector function of the antibody.

"Neutralizing antibody"or "NAb"refers to an antibody that can "neutralize" the biological effects of a foreign substance, such as a pathogen, to protect a cell or organism.

"HBV"refers to hepatitis B virus. "HDV"refers to hepatitis D virus.

"Pre-S1" is a domain located at the N-terminus of the product encoded by S gene on the surface of hepatitis B virus. The S gene contains three reading frames, namely pre-S1, pre-S2, and S, respectively. Accordingly, the S gene encodes three protein products of different lengths, among which the largest product is known as the L (large) protein, which contains the pre-S1, pre-S2 and S domains. The additional portion of the L peotein relative to the other two products (the M protein containing pre-S2 and S domains, and the S protein containing S domain only) is the N-terminal pre-S1 domain.

"A14" is a fully human monoclonal antibody targeting the Pre-S1 domain of hepatitis B virus, which was previously developed by the inventor of the present application, It can inhibit the entry of hepatitis B virus into hepatocytes and has the ability to clear hepatitis B virus and/or cells infected by hepatitis B virus through an Fc-mediated immune response. A14 is an IgG1 antibody. In addition, the antibody is also a potential inhibitor for the treatment of chronic hepatitis B. More information about the A14 antibody can be found in PCT invention disclosure documents WO2016188386A1 and WO2021013135A.

"IgG1" is a subtype (sometimes called a subclass) of the immunoglobulin IgG isotype. Different IgG subtypes have different positions and numbers of disulfide bonds.

"Antibody variant" refers to an antibody that has been modified relative to a "parent antibody". The modification includes, but is not limited to, modification of the antibody sequence and modification of the antibody glycosylation. "Parent antibody" refers to an antibody prior to modification. In some specific embodiments, the parent antibody refers to the A14 antibody.

"Fc variant" or "Fc antibody variant" refers to an antibody that has been modified in the Fc region relative to the parent antibody.

"EU numbering" is a way of numbering the amino acids of an antibody. The numbering system is based on the fact that Gerald M. Edelman *et al.* isolated and purified an IgG1 immunoglobulin and named it Eu in 1968. By determining the amino acid sequence, the positions of each amino acid were numbered, thus forming the Eu numbering system. Although the optimised systems, such as Kabat, Chothia and IMGT systems, are currently more commonly used to encode the amino acid sequences of antibodies, the Eu numbering system is based on IgG1 antibodies and is therefore suitable for numbering the amino acid sequence of the IgG1 antibody A14 in the present invention. Therefore, EU numbering in the present invention is used to describe the location of amino acid mutations.

"Fc receptor" is a receptor found on the surface of various cells (such as effector cell), which can specifically bind to the Fc region of an antibody.

"Fcy receptor" or "FcyR" is a class of Fc receptors, all members of which belong to the immunoglobulin superfamily and are further classified into FcyRI, FcyRIIA, FcyRIIB, FcγRIIIA, and FcγRIIIB. Different members exhibit different affinities to subtypes of IgG and have different functions. Among the various humans Fcy receptors, FcyRI, FcyRIIa, FcγRIIIa, and FcγRIIIb are the "activating Fcy-receptors", while FcγRIIb is the only "inhibitory Fcγ-receptor" in the human body.

"Effector function" of an antibody, in the context of the present invention, refers primarily to an Fc-mediated effector function, including ADCC, ADCP, and the like.

"ADCC"is the abbreviation for antibody-dependent cell-mediated cytotoxicity, which is a cell-mediated immune mechanism. Specifically, an effector cell binds to the Fc fragment of an antibody that specifically binds to a target cell through a receptor, and releases cytotoxic substances to kill the target cell. ADCC is an important indicator of measuring the efficacy of an antibody.

"ADCP" is the abbreviation for antibody-dependent cellular phagocytosis.

"Afucosylation", "afucosylated modification" or "afucose" refers to the reduction or complete elimination of fucose moiety in the sugar chain of an antibody glycoprotein through modification. For example, when describing an antibody variant, "afucosylated" indicates that the antibody variant, relative to the parent antibody thereof, has fewer fucose-containing sugar chain, or even no fucose-containing sugar chains at all, and such an antibody variant may be represented by the abbreviation "afuco-" as a prefix. When describing a cell, "afucosylated" indicates that the cell, when used for the production of an antibody, is capable of devoid of fucose moiety in the sugar chain of an antibody, or "cell line of afucosylated protein " is used to describe such a production cell line.

"FUT8" refers to alpha-(1,6)-fucosyltransferase (α-(1,6)-fucosyltransferase), and its encoding gene is the *"Fut8* gene". FUT8 is involved in the glycosylation process following antibody expression, adding fucose to the oligosaccharide chain.

"FITC-LCA" refers to a fluorescein isothiocyanate (FITC)-labelled lens culinaris agglutinin (LCA). "PE-LCA" refers to phycoerythrin-labelled lens culinaris agglutinin. LCA has a strong affinity for fucose and can be used to detect fucose levels when labelled with FITC or phycoerythrin.

"Treatment" or "treating" refers to improvment, alleviation or elimination (i.e. cure) of a disease or symptoms thereof. In some cases, the treatment includes preventive treatment.

"Synergy or synergetic effect" refers to an additive or enhanced effect produced when two or more drugs are administered together, preferably an enhanced effect, that is, where the combined effect is superior to the sum of the effects when each drug is administered individually.

### Parent antibody

The parent antibody of the present invention is an antibody that specifically binds to the Pre-S1 domain of HBV. The primary difference between the parent antibody and the antibody variant lies in the amino acid sequence of the Fc region and the level of fucosylation.

In some preferred embodiments, the parent antibody of the present invention comprises the constant region of unmodified human IgG. For example, the parent antibody comprises a heavy chain constant region of unmodified human IgG, preferably the Fc region of unmodified human IgG. For example, the human IgG is human IgG1.

In some specific embodiments, the parent antibody of the present invention is an A14 antibody.

It should be understood that the parent antibody of the present invention may also comprise the same six CDR sequences (amino acid sequences as shown in SEQ ID NOs: 32 to 37) as the A14 antibody, and may even comprise the same heavy chain variable region (SEQ ID NO: 1) and light chain variable region (SEQ ID NO: 5). However, the heavy chain constant region, particularly the Fc region, is derived from human IgG1 and has not been modified in any way.

### Naming rule for mutation

In the context of the present invention, when describing a mutation contained in the Fc variant, unless otherwise specified, the sequence of the heavy chain constant region of human IgG1 as shown in SEQ ID NO: 31 is used as a reference, and the types and positions of the amino acid mutation are described according to the EU numbering system. Specifically, the heavy chain constant region of the human IgG1 is a sequence of 330 amino acids, ranging from alanine at position 118 to lysine at position 447 according to the EU numbering system, wherein positions 118 to 214 are CH1 region (corresponding to amino acid positions 1 to 97 of SEQ ID NO: 31), positions 215 to 230 are hinge region (corresponding to amino acid positions 98 to 113 of SEQ ID NO: 31), positions 231 to 340 are CH2 region (corresponding to amino acid positions 114 to 223 of SEQ ID NO: 31), and positions 341 to 447 are CH3 region (corresponding to amino acid positions 224 to 330 of SEQ ID NO: 31)

When describing the position of an amino acid mutation in the present invention, the position is notated by the position of the mutated amino acid according to EU numbering system, rather than its position number in SEQ ID NO: 31. As shown above, for the same amino acid, the number of the EU numbering will be 117 greater than the position number of the amino acid in SEQ ID NO: 31. When describing the type of amino acid mutation, the type of amino acid at the corresponding position in SEQ ID NO: 31 is listed before (to the left of) the position number as the amino acid before mutation, while the amino acid present in the mutated variant antibody is listed after (to the right of) the position number. When the same position can be mutated to different amino acids, multiple optional amino acids are separated by "/".

Based on the above rule, taking the G236A mutation of the present invention for example, it means the glycine at position 236 (according to the EU numbering) being mutated to alanine. Relative to the amino acid sequence of SEQ ID NO: 31, the mutation corresponds to the glycine at position 119 being mutated to alanine. For another example, S239D/Q/E means the serine at position 239 (according to the EU numbering) being mutated to aspartic acid, glutamine, or glutamic acid. Relative to the amino acid sequence of SEQ ID NO: 31, the mutation corresponds to the serine at position 122 being mutated to aspartic acid, glutamine, or glutamic acid.

For convenience, in the context of the present invention, abbreviations are sometimes used to denote the mutations or mutation combinations of several antibody variants relative to the parent antibody (A14), and these abbreviations are also used to notate the antibody variants themselves. The types of mutations or combinations of mutations specifically referred to by the abbreviations are shown in Table 6.

### Altering the ADCC and ADCP activities of an antibody

For therapeutic antibodies, FcyR-mediated effector functions, including ADCC and ADCP activities, are correlated with the ability of antibody-mediated elimination of target cells. The antibody variant of the present invention enhances the ADCC and/or ADCP activity of the original antibody through different means, thereby further enhancing the efficacy of the antibody.

Natural killer (NK) cells are the main immune effector cells involved in ADCC. In addition, monocytes and eosinophils can also mediate ADCC. In human body, the strength of antibody-mediated ADCC is related to many factors, such as the affinity of the antibody for the antigen, the affinity of the antibody Fc for the receptor FcγRIIIa, and the characteristics of the immune effector cells. It is an effective approach to enhance ADCC activity that modifies the glycosylation and amino acid sequence of the Fc fragment of an antibody to increase the affinity of Fc for FcγRIIIa. Studies have shown that afucosylated modification of the sugar chain structure at Asn297 in the CH2 domain of the Fc fragment of an IgG antibody can increase the affinity between the Fc fragment of an IgG antibody and FcγRIIIa on the surface of NK cells, thereby significantly enhancing the ADCC effect of the antibody and improving the clinical efficacy of the antibody.

In a mammal cell, the addition of core fucose to the oligosaccharide chain of the antibody Fc fragment is directly catalysed by the α-1,6 fucosyltransferase (FUT8) encoded by the *Fut8* gene. FUT8 transfers fucose residues from guanidine-diphospho-fucose (GDP-Fuc) to the terminal of the innermost N-acetylglucosamine (GlcNAc) of the oligosaccharide chain of the Fc fragment, thus forming the core fucose of Fc fragment oligosaccharide. The α-1,6-fucosyltransferase is a key glycosyltransferase in the fucosylation synthesis approach. To produce an antibody with enhanced ADCC effect, various methods have been empolyed to reduce the level of fucosylation modifications of an IgG antibody, such as using an antibody production cell line with *Fut8* gene knockout or low expression, or overexpressing GnTIII in an antibody production cell line through transgenesis.

Knockout of the *Fut8* gene in antibody production cell can be performed through various ways, such as small interfering RNA (siRNA), short hairpin RNA, homologous recombination and other methods known in the art. In some preferred embodiments of the present invention, the knockout of the *Fut8* gene in the antibody production cell is performed through nuclease-mediated homologous recombination. Nuclease-mediated homologous recombination includes, but is not limited to, CRISPR/Cas9, TALEN, and ZFN. In some more preferred embodiments, the knockout of the *Fut8* gene in the antibody production cell is performed through a CRISPR/Cas9 system. The specific CRISPR/Cas9 editing method is described in detail below.

In some embodiments, the antibody variant of the present invention comprises, relative to the parent antibody, an amino acid mutation in the Fc region to improve the ADCC activity. The amino acid mutation used to improve ADCC activity can be achieved by increasing the affinity of the antibody for an activating receptor (such as human FcγRIIIa), and/or by decreasing the affinity of the antibody for an inhibitory receptor (such as human FcγRIIb) to achieve an enhanced ADCC effect.

In some specific embodiments, the antibody variant of the present invention may comprise one or more amino acid mutations for improving the ADCC activity selected from the group consisting of: L235V, S239E/D/Q, F243L, R292P, Y300L, V305I, A330L, I332E, and P396L.

The antibody variant having amino acid mutation can be introduced by molecular biology methods. Specifically, the nucleotide sequence encoding the amino acid mutation-contained is synthesized and interted to a suitable construct. The construct is then introduced into a host cell for expression, thereby obtaining an antibody variant containing the amino acid mutations. The amino acid mutation used to improve other different effects as described below, such as those amino acid mutations for improving ADCP and half-life, can also be introduced using the same method.

The antibody variant can simultaneously have both a knockout of the *Fut8* gene and an amino acid mutation that improves ADCC activity, or it can have only one of these, thereby improving in mediating ADCC activity.

The ADCP activity mediated by antibodies in human body can also be adjusted through modification of the Fc region. The ADCP activity can be enhanced by increasing the affinity for the activating receptor human FcγRIIIa or decreasing the affinity for the inhibitory receptor human FcγRIIb.

One amino acid mutation that can enhance the ADCP activity is G236A, which can decrease the affinity for the inhibitory receptor FcγRIIb.

In some preferred embodiments, the antibody variant of the present invention comprises the modification that enhances both ADCC and ADCP functions, including afucosylated modification and the amino acid mutation in the Fc region. For example, the antibody variant of the present invention has, relative to the parent antibody:
(1) a reduced amount of sugar chain containing 1,6-fucose or an absence of sugar chain containing 1,6-fucose, and/or one or more amino acid modifications selected from the group consisting of: L235V, S239E/D/Q, F243L, R292P, Y300L, V305I, A330L, I332E, and P396L; and
(2) optionally, an amino acid mutation of G236A.

In some specific embodiments, the antibody variant of the present invention has the modification shown in items 3-33 of Table 6.

In some preferred embodiments, the antibody variant of the present invention has, relative to the parent antibody thereof, a mutation selected from the following groups (a) to (f):
(a) having a reduced amount of sugar chain containing 1,6-fucose or an absence of sugar chain containing 1,6-fucose;
(b) having a reduced amount of sugar chain containing 1,6-fucose or an absence of sugar chain containing 1,6-fucose, and having an amino acid mutation of G236A (GA mutation);
(c) having amino acid mutations of G236A and S239D (GASD mutation);
(d) having mutations of F243L, R292P, L235V, Y300L, and P396L (FLRPLVYLPL mutation);
(e) having mutations of F243L, R292P, Y300L, V305I, and P396L (FLRPYLVIPL mutation); and
(f) having mutations of G236A and I332E (GAIE mutation).

In a particularly preferred embodiment, the antibody variant of the present invention has, relative to the parent antibody thereof, a reduced amount of sugar chain containing 1,6-fucose or an absence of sugar chain containing 1,6-fucose, and an amino acid mutation of G236A.

### Altering half-life of antibody

As a protein molecule, it is known that the half-life of antibody can be altered through various methods.

In some specific embodiments of the present invention, the half-life is altered through changing the affinity of the antibody for the FcRn receptor. In some specific embodiments, the affinity of the resulting antibody variant for the FcRn receptor is enhanced by introducing an amino acid mutation in the Fc region of the antibody, thereby prolonging the half-life.

In some specific embodiments, the antibody variant of the present invention comprises, relative to the parent antibody thereof, one or more amino acid mutations selected from the group consisting of: M252Y, S254T, T256E, M428L, and N434S.

In some more specific embodiments, the antibody variant of the present invention comprises, relative to the parent antibody thereof, an amino acid mutation selected from the following group (g) or (h):
(g) amino acid mutations of M428L and N434S (LS mutation); and
(h) amino acid mutations of M252Y, S254T, and T256 (YTE mutation).

### Combined modification

In some preferred embodiments, the antibody of the present invention has simultaneously both an improved Fc-mediated effector function and a prolonged half-life.

In some specific embodiments, the antibody variant of the present invention comprises, relative to the parent antibody thereof:
(1) a reduced amount of sugar chain containing 1,6-fucose or an absence of sugar chain containing 1,6-fucose, and/or one or more amino acid mutations selected from the group consisting of: L235V, S239E/D/Q, F243L, R292P, Y300L, V305I, A330L, I332E, and P396L;
(2) optionally, an amino acid mutation of G236A; and
(3) optionally, one or more amino acid mutations selected from the group cosisting of: M252Y, S254T, T256E, M428L, and N434S.

In some specific embodiments, the antibody variant of the present invention has, relative to the parent antibody thereof, one set of mutation selected from (a) to (f) and one mutation selected from (g) or (h), or differs relative to the parent antibody only by one set of mutation selected from (a) to (f) and one mutation selected from (g) or (h):
(a) having a reduced amount of sugar chain containing 1,6-fucose or an absence of sugar chain containing 1,6-fucose;
(b) having a reduced amount of sugar chain containing 1,6-fucose or an absence of sugar chain containing 1,6-fucose, and an amino acid mutation of G236A (GA mutation);
(c) having amino acid mutations of G236A and S239D (GASD mutation);
(d) having amino acid mutations of F243L, R292P, L235V, Y300L, and P396L (FLRPLVYLPL mutation);
(e) having amino acid mutations of F243L, R292P, Y300L, V305I, and P396L (FLRPYLVIPL mutation);
(f) having amino acid mutations of G236A and I332E (GAIE mutation);
(g) having amino acid mutations of M428L and N434S (LS mutation); and
(h) having amino acid mutations of M252Y, S254T, and T256E (YTE mutation).

In the most specific embodiment, the antibody variant of the present invention has, relative to the parent antibody thereof, a reduced amount of sugar chain containing 1,6-fucose or an absence of sugar chain containing 1,6-fucose, and an amino acid mutation of G236A, M428L, and N434S.

In some preferred embodiments, although the antibody variant of the present invention has undergone one or more modifications relative to the parent antibody, it retains the comparable affinity for the Pre-S1 region of the hepatitis B virus and/or a virulence activity to the parent antibody. For example, the antibody variant of the present invention alters, relative to the parent antibody, only the Fc region without altering the variable region and the complementary determining region (CDR), and therefore it retains the comparable binding affinity for the hepatitis B virus, specifically the Pre-S1 protein to the parent antibody. For example, the antibody variant of the present invention has, relative to the parent antibody, a comparable IC₅₀ value in an *in vitro* antiviral activity assay. "Comparable" activity or value refers to being essentially the same or similar. For example, in an *in vitro* antiviral activity assay, "comparable activity to the parent antibody" means that the difference between the relative activity (IC₅₀ value) of the antibody variant of the present invention and the parent antibody thereof is not more than 20%.

### Production cell line

In the present invention, a mammalian cell line commonly used in the field for antibody production can be used to produce the antibody of the present invention. The cell particularly suitable for the present invention is that which can serve as a host cell for the development and production of recombinant protein drugs in the biopharmaceutical industry. In some preferred embodiments, a knockout of the *Fut8* gene is required for the production cell line of the present invention.

In some specific embodiments, the production cell line suitable for the present invention includes, but is not limited to, 293F, CHO-K1, CHOZN GS^{-/-}, and CHOK1Q. These engineered cells can all be used for the development and production in the pharmaceutical industry, with a cell doubling time maintained between 12 and 40 hours and a cell viability of ≥ 90%.

The 293F cell is derived from primary human embryonic kidney cells, which has a high transfection efficiency and can be cultured in suspension in serum-free medium with high protein expression. The 293F cell proliferates rapidly and can be cultured at high density.

CHO-K1, CHOZN GS^{-/-}, and CHOK1Q are all adult Chinese hamster ovary cells (CHO). CHO-K1 is an unmodified wild type CHO cell. CHOZN GS^{-/-} cell line has been knocked out for the glutamine synthetase gene using ZFN (Zinc Finger Nuclease) technology, which renders the cell line dependent on exogenous glutamine supplementation for survival, and thus allows the target clone to be highly expressed without the need for additional MTX or MSX pressure throughout the whole screening process. CHOK1Q is a wild-type CHO-K1 cell that has not been genetically modified, but it has been adapted for suspension culture, high osmolarity, high ammonium ion, and high lactate.

### Preparation method

As described above, knockout of the *Fut8* gene in an antibody production cell line can be achieved through various ways. The CRISPR/Cas method used in the present invention has several advantages over other methods, including ease of operation, high targeting precision, high editing efficiency, no exogenous DNA insertion into the host genome, and high safety.

CRISPR/Cas9 gene editing technology uses guide RNA (sgRNA) to direct the Cas9 endonuclease to cleave the target double-stranded DNA in the genome, causing a double-stranded break in the target sequence, and then generating substitutions, deletions, insertions, etc. in the genome under the action of intracellular DNA repair mechanism, thereby achieving the knockout of the target gene. The CRISPR/Cas9 system has been widely used for gene editing in various organisms. We utilized CRISPR/Cas9 technology to target and knock out the *Fut8* gene in 293F, CHOZN GS^{-/-}, CHOK1, or CHOK1Q cells, thereby obtaining a cell capable of producing an afucosylated antibody, and increasing the ADCC activity of the antibody.

Using a 293F cell as the production cell line, the *Fut8* gene can be knocked out using a nucleotide sequence of SEQ ID NO: 10 as sgRNA.

Using a CHO-K1 and derived cell lines thereof as the production cell lines, the *Fut8* gene can be knocked out using a nucleotide sequence selected from the group consisting of SEQ ID NO: 19, SEQ ID NO: 20, and SEQ ID NO: 21 as sgRNA. Preferably, sgRNA with nucleotide sequence of SEQ ID NO: 20 is used.

### Use

Unless otherwise specified, the antibody or antibody variant of the present invention is intended for human use.

The antibody or antibody variant of the present invention can be used for the treatment or prevention of hepatitis B, in particular for the treatment or prevention of chronic hepatitis B.

The antibody or antibody variant of the present invention may be used as an adjuvant treatment for the treatment of hepatitis B, serving as an adjunct to first-line therapeutic agents to further improve therapeutic efficacy.

The antibody of the present invention can be used in combination with other treatments for hepatitis B. Anti-hepatitis B virus drugs that can be used in combination with the antibody of the present invention include an immunomodulator, such as interferon, thymosin-α1, and cytokines; a nucleot(s)ide analogue, such as entecavir, telbivudine, adefovir or adefovir dipivoxil, tenofovir, and lamivudine. For example, it can be used in combination with currently clinically approved anti-hepatitis B virus drugs. In some specific embodiments, the antibody or antibody variant of the present invention can be used in combination with entecavir.

In some cases, the antibody or antibody variant of the present invention may also be used for the treatment or prevention of hepatitis D.

The antibody or antibody variant of the present invention may be administered to a subject by conventional routes of administration. In some preferred embodiments, the antibody of the present invention is administered to the subject via a parenteral route, preferably intravenous infusion or subcutaneous injection.

The present invention also relates to the following items:
1. An antibody variant specifically binding to a Pre-S1 domain of hepatitis B virus (HBV), having enhanced Fc-mediated effector function compared to a parent antibody thereof.
2. The antibody variant according to item 1, wherein the Fc-mediated effector function is ADCC and/or ADCP activity.
3. The antibody variant according to item 1 or 2, having enhanced affinity for an activating Fcy receptor and/or reduced affinity for an inhibitory Fcy receptor.
4. The antibody variant according to any one of items 1 to 3, wherein the antibody variant and the parent antibody are IgG1 antibodies.
5. The antibody variant according to any one of items 1 to 4, wherein the antibody has, relative to the parent antibody thereof, a characteristic selected from the following groups:
   (1) a reduced amount of or an absence of sugar chain containing 1,6-fucose, and/or one or more amino acid modifications selected from the group consisting of: L235V, S239E/D/Q, F243L, R292P, Y300L, V305I, A330L, I332E, and P396L; and
   (2) optionally, an amino acid mutation of G236A.
6. The antibody variant according to item 5, having relative to the parent antibody the mutations shown in items 3 to 33 of Table 6.
7. The antibody variant according to item 5, having relative to the parent antibody a mutation selected from any of the following groups (a) to (f):
   (a) having a reduced amount of sugar chain containing 1,6-fucose or an absence of sugar chain containing 1,6-fucose;
   (b) having a reduced amount of sugar chain containing 1,6-fucose or an absence of sugar chain containing 1,6-fucose, and having an amino acid mutation of G236A (GA mutation);
   (c) having amino acid mutations of G236A and S239D (GASD mutation);
   (d) having mutations of F243L, R292P, L235V, Y300L, and P396L (FLRPLVYLPL mutation);
   (e) having mutations of F243L, R292P, Y300L, V305I, and P396L (FLRPYLVIPL mutation); and
   (f) having mutations of G236A and I332E (GAIE mutation).
8. The antibody variant according to item 7, having a reduced amount of sugar chain containing 1,6-fucose or an absence of sugar chain containing 1,6-fucose, and having the amino acid mutation of G236A (GA mutation).
9. The antibody variant according to any one of items 5 to 8, wherein the reduction or absence of the sugar chain comprising 1,6-fucose is achieved by knocking out the *Fut8* gene in a host cell used to produce the antibody variant.
10. The antibody variant according to any one of items 1 to 9, having a prolonged half-life relative to the parent antibody.
11. The antibody variant according to claim 10, comprising relative to the parent antibody one or more amino acid mutations selected from the group consisting of: M252Y, S254T, T256E, M428L, and N434S.
12. The antibody variant according to claim 11, comprising relative to the parent antibody an amino acid mutation selected from the following group (g) or (h):
   (g) amino acid mutations of M428L and N434S (LS mutation); and
   (h) amino acid mutations of M252Y, S254T, and T256E (YTE mutation).
13. The antibody variant according to any one of items 1 to 12, having relative to the parent antibody:
   (1) a reduced amount of sugar chain containing 1,6-fucose or an absence of sugar chain containing 1,6-fucose, and/or one or more amino acid mutations selected from the group consisting of: L235V, S239E/D/Q, F243L, R292P, Y300L, V305I, A330L, I332E, and P396L;
   (2) optionally, an amino acid mutation of G236A; and
   (3) optionally, one or more amino acid mutations selected from the group consisting of: M252Y, S254T, T256E, M428L, and N434S.
14. The antibody variant according to item 13, relative to the parent antibody thereof, having one mutation selected from (a) to (f) and one mutation selected from (g) or (h):
   (a) having a reduced amount of sugar chain containing 1,6-fucose or an absence of sugar chain containing 1,6-fucose;
   (b) having a reduced amount of sugar chain containing 1,6-fucose or an absence of sugar chain containing 1,6-fucose, and having an amino acid mutation of G236A (GA mutation);
   (c) having amino acid mutations of G236A and S239D (GASD mutation);
   (d) having amino acid mutations of F243L, R292P, L235V, Y300L, and P396L (FLRPLVYLPL mutation);
   (e) having amino acid mutations of F243L, R292P, Y300L, V305I, and P396L (FLRPYLVIPL mutation);
   (f) having amino acid mutations of G236A and I332E (GAIE mutation);
   (g) amino acid mutations of M428L and N434S (LS mutation); and
   (h) amino acid mutations of M252Y, S254T, and T256 (YTE mutation).
15. The antibody variant according to item 14, having relative to the parent antibody a reduced amount of sugar chain containing 1,6-fucose or an absence of sugar chain containing 1,6-fucose, and amino acid mutations of G236A, M428L, and N434S.
16. The antibody variant according to any one of items 1 to 15, wherein the parent antibody and the antibody variant have CDR sequences as shown in SEQ ID NOs: 32 to 37.
17. The antibody variant according to item 16, wherein the parent antibody is an A14 antibody, having a heavy chain variable region sequence as shown in SEQ ID NO: 1, a heavy chain constant region sequence as shown in SEQ ID NO: 38, a light chain variable region sequence as shown in SEQ ID NO: 5, and a light chain constant region sequence as shown in SEQ ID NO: 6.
18. The antibody variant according to any one according to items 1 to 17, having a similar anti-hepatitis B virus activity compared to the parent antibody, for example, having a similar IC50.
19. An antibody specifically binding to a Pre-S1 domain of hepatitis B virus (HBV), comprising:
   (1) three heavy chain CDR sequences as shown in SEQ ID NOs: 32, 33, and 34, and three light chain CDR sequences as shown in SEQ ID NOs: 35, 36, and 37; and/or
   (2) a heavy chain constant region comprising an amino acid sequencea as shown in SEQ ID NO: 2.
20. The antibody according to item 19, comprsing:
   (1) a heavy chain variable region sequence as shown in SEQ ID NO: 1 and a light chain variable region sequence as shown in SEQ ID NO: 5; and/or
   (2) a heavy chain constant region comprising an amino acid sequencea as shown in SEQ ID NO: 2.
21.An isolated nucleic acid molecule, comprising a nucleotide sequence encoding the antibody variant according to any one of items 1 to 18 or the antibody according to any one of items 19 to 20.
22. The isolated nucleic acid molecule according to item 21, comprising a nucleotide sequence as shown in SEQ ID NO: 4 to encode a heavy chain constant region.
23. An expression vector, comprising the isolated nucleic acid molecule according to item 21 or 22.
24. A host cell, comprising the isolated nucleic acid molecule according to item 21 or 22 or the expression vector according to item 23.
25. A mammalian cell, characterised in that FUT8 is inactivated in the cell and the isolated nucleic acid molecule according to item 21 or 22 or the expression vector according to item 23 is introduced into the cell.
26. The mammalian cell according to item 25, wherein FUT8 is inactivated by knocking out the *Fut8* gene of the cell.
27. The mammalian cell according to item 25 or 26, being selected from the group consisting of: a 293F cell, a CHOK1 cell, a CHOZN GS-/- cell, and a CHO K1Q cell.
28. The mammalian cell according to item 25, which is a 293F cell.
29. A method for preparing an antibody, comprising the following steps:
   (1) culturing the mammalian cell according to any one of items 25 to 28 in a medium to obtain a culture comprising the antibody; and
   (2) recovering the antibody from the culture.
30. A method for preparing a mammalian cell with *Fut8* gene knockout, comprising introducing into the mammalian cell:
   (a) a gRNA targeting *Fut8* and a coding sequence of a Cas9 nuclease, or
   (b) a complex of a gRNA targeting *Fut8* and a Cas9 nuclease,
      wherein (b) is preferably introduced.
31. The method according to item 30, wherein the mammalian cell is selected from the group consisting of: a 293F cell, a CHOK1 cell, a CHOZN GS-/- cell, and a CHO K1Q cell.
32. The method according to claim 31, wherein the nucleotide sequence of the gRNA comprises a nucleotide sequence selected from the following groups for targeting the *Fut8* gene:
   nucleotide positions 1 to 20 of the nucleotide sequence as shown in SEQ ID NO: 10,
   nucleotide positions 1 to 20 of the nucleotide sequence as shown in SEQ ID NO: 19,
   nucleotide positions 1 to 20 of the nucleotide sequence as shown in SEQ ID NO: 20,
   nucleotide positions 1 to 20 of the nucleotide sequence as shown in SEQ ID NO: 21, or
   nucleotide positions 21 to 40 of the nucleotide sequence as shown in SEQ ID NO: 39.
33. The method according to item 32, wherein the nucleotide sequence of the sgRNA is selected from a nucleotide sequence as shown in SEQ ID NO: 10, SEQ ID NO: 19, SEQ ID NO: 20, SEQ ID NO: 21 or SEQ ID NO: 39.
34. A mammalian cell being prepared by the method according to any one of items 30 to 33.
35. An sgRNA for knockout of the *Fut8* gene, having a nucleotide sequence selected from a nucleotide sequence as shown in SEQ ID NO: 10, SEQ ID NO: 19, SEQ ID NO: 20, SEQ ID NO: 21 or SEQ ID NO: 39.
36. The sgRNA according to item 35, which is used to knock out the *Fut8* gene in a 293F cell and has a nucleotide sequence as shown in SEQ ID NO: 10.
37. The sgRNA according to item 35, which is used to knock out the *Fut8* gene in a CHO-K1 cell and derived cells thereof and has a nucleotide sequence as shown in SEQ ID NO: 19, SEQ ID NO: 20, SEQ ID NO: 21 or SEQ ID NO: 39.
38. A method for preparing an antibody, comprising the following steps:
   (1) introducing the isolated nucleic acid molecule according to item 21 or 22 or the expression vector according to item 23 into the mammalian cell according to item 34;
   (2) culturing the mammalian cell obtained from step (1) to produce a culture comprising the antibody; and
   (3) recovering the antibody from the culture of step (2).
39. An antibody prepared by the method according to item 29 or 38.
40. A pharmaceutical composition, comprising the antibody variant according to any one of items 1 to 18, the antibody according to any one of items 19 to 20 or 39, and a pharmaceutically acceptable carrier.
41. Use of the antibody variant according to any one of items 1 to 18, or the antibody according to any one of items 19 to 20 or 39, in the preparation of a medicament, wherein the medicament is used in a subject in need thereof for the treatment of hepatitis B or D virus infection or disease caused by the infection, or for eliciting the effects of ADCC.
42. The use according to item 41, wherein the subject is selected from the group consisting of: a subject with confirmed infection with hepatitis B virus or hepatitis D virus, a subject being previously exposed to hepatitis B virus or hepatitis D virus, a subject at high risk of exposure to hepatitis B virus or hepatitis D virus, or a subject with chronic hepatitis B.
43. The use according to item 41 or 42, wherein the medicament is used for the treatment of chronic hepatitis B.
44. A method for treating hepatitis B or D virus infection, or a disease caused by the infection, or for inducing an ADCC effect in a subject, comprising administering to the subject a therapeutically effective amount of the antibody variant according to any one of items 1 to 18, the antibody according to any one of items 19 to 20 or 39, or the pharmaceutical composition according to item 40.
46. The method according to item 44 or 45, wherein the subject is selected from the group consisting of: a subject with confirmed infection with hepatitis B virus or hepatitis D virus, a subject being previously exposed to hepatitis B virus or hepatitis D virus, a subject at high risk of exposure to hepatitis B virus or hepatitis D virus, or a subject with chronic hepatitis B.
47. The method according to any one of items 44 to 46, wherein the antibody variant or antibody is administered by intravenous infusion or subcutaneous injection.
48. The method according to any one of items 44 to 47, wherein the method comprises administering to the subject another medicament, and said another medicament is used for the treatment or prevention of a disease caused by hepatitis B virus (HBV) infection, in particular a chronic disease caused by hepatitis B virus (HBV) infection.
49. The method according to item 48, wherein said another medicament is an immunomodulator or a nucleoside analogue.
50. A pharmaceutical composition for use in the treatment of hepatitis B or D virus infection or a disease caused by the infections, comprising: (a) the antibody variant according to any one of items 1 to 18, the antibody according to any one of items 19 to 20 or 39; and (b) another medicament for use in the treatment or prevention of diseases caused by hepatitis B virus (HBV) infection, in particular a chronic disease caused by hepatitis B virus (HBV) infection.
51. The pharmaceutical composition according to item 50, wherein said another antiviral medicament is an immunomodulator or a nucleoside analogue.

### Examples

To provide a more comprehensive understanding and application of the present invention, the following detailed description will be made with reference to examples and drawings. The examples are intended to illustrate the present invention only, and are not intended to limit the scope of the present invention. The scope of the present invention is specifically claimed by the appended claims.

### Example 1. Establishment of a Cell Line for Producing Afucosylated Protein

This example described the establishment of a cell line for producing afucosylated protein. Specifically, the *Fut8* gene in the 293F cell line was knocked out using the Crispr/Cas9 nuclease editing system, such that the antibody produced by this edited cell lacked the fucosylation modification.

First, the sgRNAs targeting the target gene required for the Crispr/Cas9 system were designed. Using the sgRNA design website http://crispr.dbcls.jp/, two sgRNA sequences were designed to target the *Fut8* gene (NM_178155.2) in 293F cells (purchased from Beijing Sino Biological, Inc.). The specific sequences were as shown in SEQ ID NO:9 and SEQ ID NO: 10 below. In the following sequences, the sgRNA-targeted sequences were underlined, and the PAM sequences (protospacer adjacent motif) were in bold.
sgFut8-1: ATTCCAAGATGAGTGTTCGC**TGG** (SEQ ID NO: 9), and
sgFut8-2: CTCGTACAAGTCGATCTGCG**AGG** (SEQ ID NO: 10).

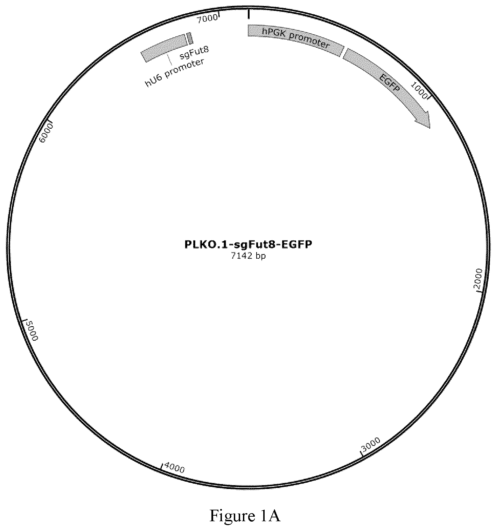

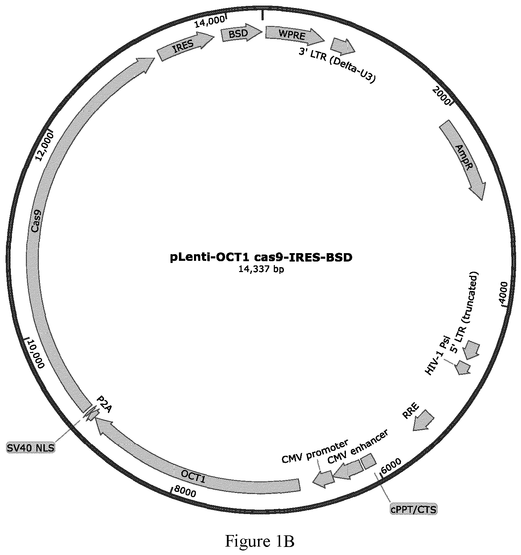

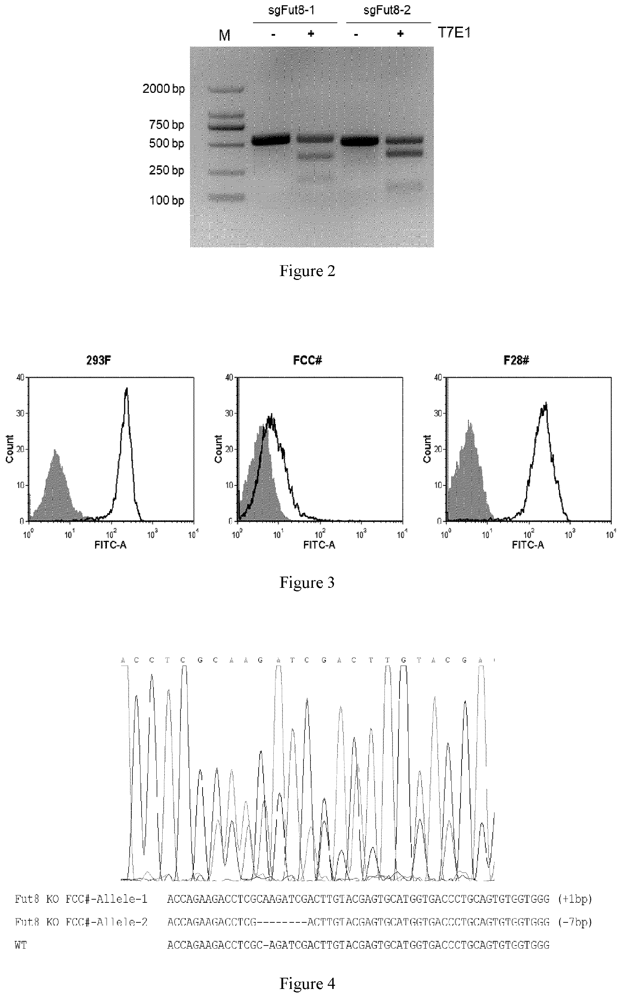

Subsequently, two pairs of primers (sgFut8-1-F1 and sgFut8-1-R1; sgFut8-2-F1 and sgFut8-2-R1, see Table 1) were synthesised. After synthesis by *in vitro* annealing, the double-stranded products were cloned into the BfuAI-cleaved pLenti-sgRNA-EGFP vector (purchased from Addgene), and the plasmids pLenti-sgFut8-1-EGFP and pLenti-sgFut8-2-EGFP expressing sgRNA were obtained, respectively (Figure 1A), and then co-transfected with the plasmid pLenti-OCT1-cas9-IRES-BSD expressing Cas9 (purchased from Addgene) into 293F cells (purchased from Beijing Sino Biological, Inc.) using PEI (Figure 1B). At 48 hours post-transfection, EGFP-positive cells were sorted through flow cytometry and were subsequently maintained to be cultured until 7 days post-transfection. The cells were then collected for T7E1 (NEB) validation. In the T7E1 assay, two pairs of primers (T7-sgFut8-1-F1 and T7-sgFut8-1-R1; T7-sgFut8-2-F1 and T7-sgFut8-2-R1) shown in Table 1 were used for the two sgRNAs, respectively. Given that T7E1 endonuclease can recognize mismatched bases in DNA and cleave the identified site, the T7E1 assay can be used following gene editing to conduct a semi-quantitative assay of gene editing efficiency, therby determining the efficiency of gene editing. The results of the assay were shown in Figure 2. As shown in Figure 2, the two designed sgRNAs were confirmed to be efficiently edit the *Fut8* gene in the cells.

**Table 1. Primer Sequences for Knocking Out the Fut8 Gene in 293F Cells**

| **Primer Name** | **Primer Sequence (5'-3')** | **Application** | **Note** | **SEQ ID NO:** |
|---|---|---|---|---|
| sgFut8-1-F1 | *CACCG*ATTCCAAGATGAGTGTTCGC | Cloning into pLenti-sgRNA-EGFP vector for expressing sgRNA | Italic represents BfuAI-cleaved end and underlined represents sgRNA-targeted sequence. | 11 |
| sgFut8-1-R1 | *AAAC*GCGAACACTCATCTTGGAATC | | | 12 |
| T7-sgFut8-1-F1 | CCTACTTGAGGGTGAAAGGTGGGAGGA | Primers for amplifying the sgRNA-targeted region of the Fut8 gene for the T7E1 assay | NA | 15 |
| T7-sgFut8-1-R1 | AGTATGTTCCATGAAGGTCTACCTAG | | | 16 |

Next, the monoclonal cell was obtained from the T7E1-validated cell through the limiting dilution method, and the clonal cell was subjected to a cell-binding assay based on flow cytometry (FACS). The monoclonal cell was verified to be a gene-edited monoclonal cell through functional phenotypic analysis. Lens culinaris agglutinin (LCA) is a lectin that specifically recognises and binds to polysaccharides with fucosylation, and fluorescein isothiocyanate (FTIC)-labelled LCA (FITC-LCA) can be used to detect fucosylation and the level of fucosylation of cell-surface proteins. The results of the FACS were shown in Figure 3. As shown in Figure 3, the unedited 293F wild-type cell exhibited robust binding to FITC-LCA, with the highest FITC signal value; whereas the successfully edited cell, such as the FCC# cell clone, exhibited significantly reduced binding to LCA. For another example, the F28# cell clone showed no difference in the ability to bind LCA compared to the wild-type cell, indicating that the F28# cell was an unsuccessful edited cell. Further Sanger sequencing of the FCC# cell was performed to determine the specific form of gene editing. The results showed that the FCC# cell was a cell of homozygous knockout of *Fut8* (Figure 4).

The above results indicated that the FCC# clone obtained is a 293F cell with a stable knockout of the *Fut8* gene (referred to as "FCC#-293F cell"), which can be used for the expression of an afucosylated antibody. The cell may be used as a host cell for the production of an afucosylated antibody.

### Example 2. Establishment of Afucosylated CHOK1, CHOZNGS-/-, and CHO K1Q Cell Lines 2.1 sgRNA design and validation

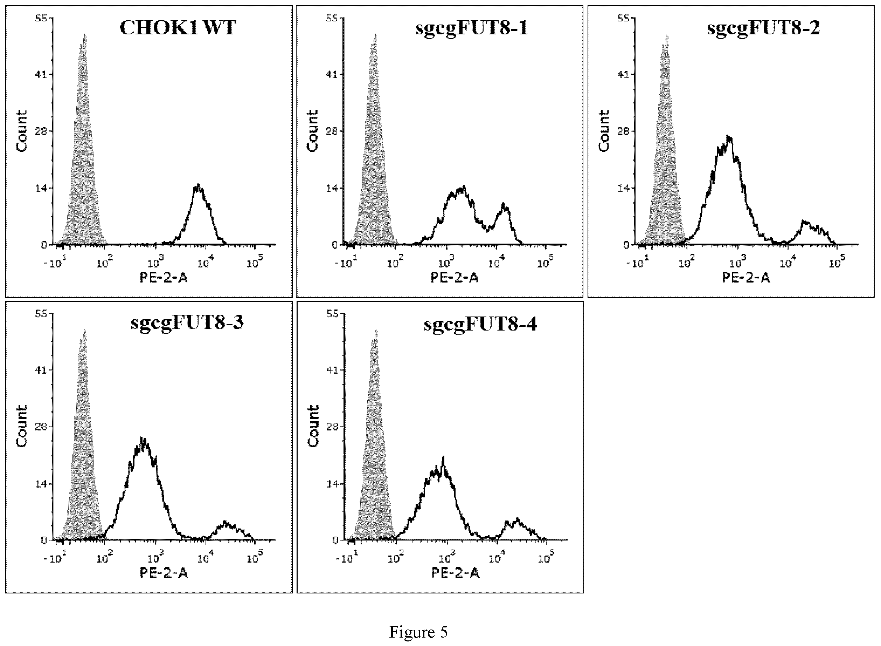

Using the sgRNA online design website http://crispr.dbcls.jp/, four sgRNA sequences targeting the *Fut8* gene were designed based on the gene sequence of CHO-K1 cells (purchased from Cell Culture Center, Institute of Basic Medical Sciences, Chinese Academy of Medical Sciences) published in the NCBI database (Gene ID: 100751648, NCBI Reference number: NM_003613860) (see Table 2). Using the method as described in Example 1, the sgRNA fragments were ligated into the pLenti-sgRNA-EGFP vector, and then respectively co-transfected with the plasmid pLenti-OCT1-Cas9-IRES-BSD expressing Cas9 into CHO-K1 cells (purchased from the National Infrastructure of Cell Line Resource) using PEI. Two days post-transfection, EGFP-positive cells (i.e. cells co-expressing sgRNA and Cas9) were screened through flow cytometry, and subsequently maintained to be cultured for 5 days. Thereafter the cells were subjected to a cell-binding assay using Rhodamine (Rho)-labeled LCA. The results were shown in Figure 5, all four designed sgRNAs effectively edited the *Fut8* gene in CHO-K1 cells, and the ability of the cell co-expressing sgcgFut8 and Cas9 to bind Rho-LCA was greatly reduced, indicating that the fucose level of cell surface proteins was significantly reduced. This result demonstrated the successful editing of the *Fut8* gene, effectively silencing the expression of the FUT8 protein.

**Table 2. sgRNAs targeted sequences to target the Fut8 gene in CHO-K1 cell**

| **sgRNA Name** | **Sequence (5'-3')** | **Targeting region** | **Note** | **SEQ ID NO** |
|---|---|---|---|---|
| sgcgFUT8-1 | ATTCCAAGATGAGTGTTCGC**TGG** | Exon 7 | Underlined represents sgRNA-targeted sequences, and bold represents PAM sequences. | 19 |
| sgcgFUT8-2 | CTCTCAGGAGTCGATCTGCA**AGG** | Exon 8 | | 20 |
| sgcgFUT8-3 | ACTTGATCCGTCCACAACCT**TGG** | Exon 8 | | 21 |

### 2.2 Establishment of afucosylated CHOZNGS-/- stable cell line

To establish an afucosylated CHOZNGS-/- stable cell line, one sgRNA (sgcgFut8-2) out of the four sgRNAs validated as described in section 2.1 was selected for gene editing. In order to deliver sgRNA/Cas9 into cells, a DNA-free, viral vector-free, and transfection reagent-free approach was used. The information of the primer sequences used in the study can be found in Table 3.

**Table 3. List of primer sequences**

| **Primer Name** | **Primer Sequence (5'-3')** | **Application** | **SEQ ID NO** |
|---|---|---|---|
| T7-sgcgFut8-2 | ttaatacgactcactataggctctcaggagtcgatctgca | Preparation of sgDNA templates | 23 |
| sgRNA-R1 | gctatttctagctctaaaactgcagatcgactcctgagag | | 24 |
| crRNA-F2 | gttttagagctagaaatagcaagttaaaataaggct | | 25 |
| crRNA-R2 | tttttcaagttgataacggactagccttattttaactt | | 26 |
| crRNA-F3 | gttatcaacttgaaaaagtggcaccgagtcgg | | 27 |
| T7-sgRNA-R | aaaaaaagcaccgactcggtgccac | | 28 |
| T7E1-sgcgFut8-2F | agagttgctgtgaaggtacaagtactc | T7E1 Assay | 29 |
| T7E1-sgcgFut8-2R | atttgctctgctgccctaactgaacaa | | 30 |

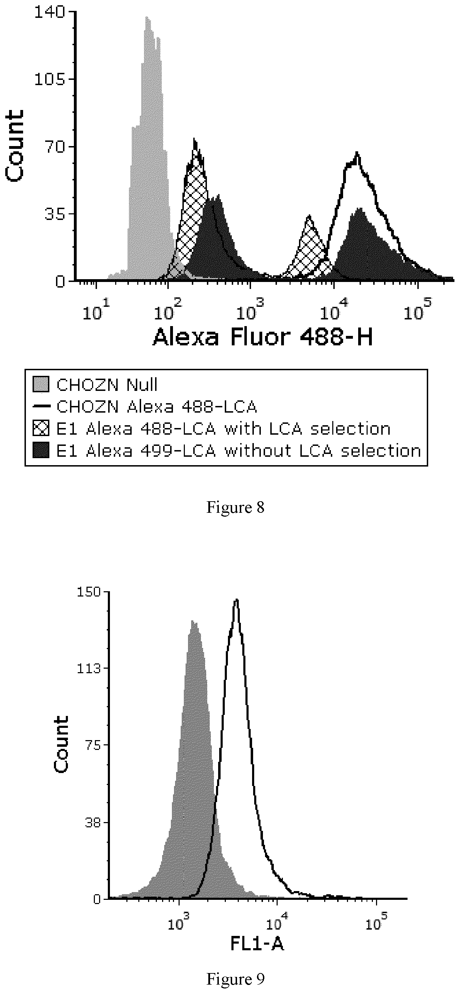

A gRNA/Cas9 ribonucleoprotein (RNP) complex was formed by co-incubating *in vitro* transcriptionally synthesised sgRNA with Cas9 protein containing a signal peptide. This RNP complex was then electrotransfected into CHOZNGS-/- cells (purchased from Merck, Batch No: 2113-53667) to enable targeted cleavage of the DNA double strand in the cellular genome for editing the *Fut8* gene. In contrast to the method of delivering sgRNA and Cas9 coding sequences by plasmid in Example 1, the delivery method of delivering RNP complexes via electrotransfection offers the several advantages: ease of operation, high specificity for gene editing, no risk of DNA insertion mutations and no cytotoxicity induced by the transfection reagents, as shown in Figure 8. LCA, as a lectin that specifically binds fucose, is endocytosed into the cell following binding to afucosylated proteins on the cell membrane surface, ultimately leading to cell death. Based on this principle, LCA pressure selection was combined to improve the success rate of obtaining the *Fut8* gene knockout cells.

### 2.2.1 Preparation of sgDNA template

The template sgDNA sequence (SEQ ID NO: 39) was designed as follows: 
TTAATACGACTCACTATAggCTCTCAGGAGTCGATCTGCAGTTTTAGAGCTAGAA
ATAGCAAGTTAAAATAAGGCTAGTCCGTTATCAACTTGAAAAAGTGGCACCGAGTCG
GTGCTTTTTTT
 wherein underlined represented the sequence of the T7 promoter, italic represented gRNA targeted sequence, and normal font represented gRNA conserved scaffold sequences.

The Overlap PCR reaction system for preparing a sgDNA template above was shown in Table 4 below. The PCR reaction conditions were as follows: pre-denaturation at 98°C for 2 min; then denaturation at 98°C for 10 s, annealing at 60°C for 10 s, and extension at 72°C for 10 s for a total of 35 cycles; and finally, extension at 72°C for another 4 min. The PCR products were analyzed by a 2% agarose gel electrophoresis after PCR reaction. The gel was cut to recover the product expected to be a 123 bp fragment. The correct synthesis of the sgDNA template was then identified through T7-sgcgFut8-2 sequencing.

**Table 4. Overlap PCR reaction system for preparing sgDNA template**

| **Reaction component** | **Volume** |
|---|---|
| T7- sgcgFut8-2 (10µM) | 8µl |
| sgRNA-R1(10µM) | 4µl |
| crRNA-F2(10µM) | 4µl |
| crRNA-R2(10µM) | 4µl |
| crRNA-F3(10µM) | 4µl |
| T7-sgRNA-R(10µM) | 8µl |
| primerSTAR HS (premix) | 100µl |
| H₂O | Added to 200µl |

### 2.2.2 In vitro transcription for gRNA by T7

The following reaction system was prepared using sgDNA as a template, according to the instructions of the T7 *In Vitro* Transcription Kit (NEB) (see Table 5) and incubated at 72°C for 16 hours. The gRNA product obtained after transcription was purified to obtain gRNA.

**Table 5. In Vitro Transcription for preparing gRNA by T7**

| **Reaction component** | **Volume** |
|---|---|
| sgDNA- sgcgFut8-2 (1µg) | 18.3µl |
| 10 × reaction buffer | 1.5µl |
| NTP | Each 1.5µl |
| T7 RNA polymerase Mix | 1.5µl |
| Nuclease free water | Added to 20µl |

### 2.2.3 Preparation of gRNA/Cas9 complex

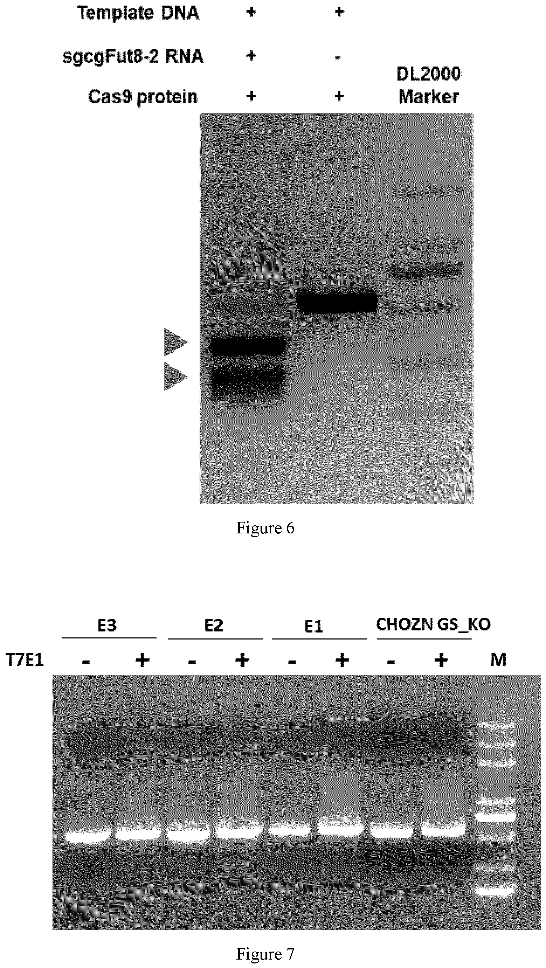

The above prepared sgcgFut8-2 gRNA was incubated with EnGen Cas9-NLS (20uM, NEB) protein at a molar ratio of 2:1 for 10 to 15 min at room temperature to form a gRNA/Cas9 complex. *In vitro* CRISPR/Cas9 cleavage assay demonstrated (Figure 6) that the CHOK1 Fut8 DNA fragment containing the sgcgFut8-2 targeted sequence could be efficiently cleaved following the formation of the complex of the synthesised sgcgFut8-2 gRNA with the EnGen Cas9-NLS. This indicated that the established gene editing system could function effectively.

### 2.2.4 Screening and establishment of Fut8 knockout CHOZN GS-/- cell lines by electrotransfection

Next, the gRNA/Cas9 complex prepared in section 2.2.3 was electrotransfected into CHOZN GS^{-/-} cells using a Gene Pulser Xcell Electroporator. A total of three independent experiments were performed.

One week following electrotransfection, some cells were collected and genomic DNA thereof was extracted. The genomic DNA fragment containing the sgcgFut8 targeted region was amplified by PCR using the T7E1-sgcgFut8-2F and T7E1-sgcgFut8-2R primers. T7E1 digestion was then performed to verify the efficiency of sgcgFut8-2 gRNA/Cas9-mediated gene editing. The results demonstrated that sgcgFut8-2 gRNA successfully directed Cas9 to cleave the *Fut8* gene in CHOZN GS-/- cells (Figure 7).

To enrich the cells with editing of the *Fut8* genes, LCA pressure selection was performed. Twelve days after selection, the ability of cells to bind FITC-LCA was analyzed through FACS. The results demonstrated that wild-type CHOZN GS-/- cells bound FITC-LCA with high affinity; while some cell populations in the sgcgFut8-2 gRNA/Cas9-electransfected cells displayed a loss of capacity to bind FITC-LCA. The results indicated the existence of successful editing of the *Fut8* gene. After further LCA pressure selection, the number of cells binding FITC-LCA was significantly reduced, indicating that the cells with successful editing of the *Fut8* gene were effectively enriched (Figure 8).

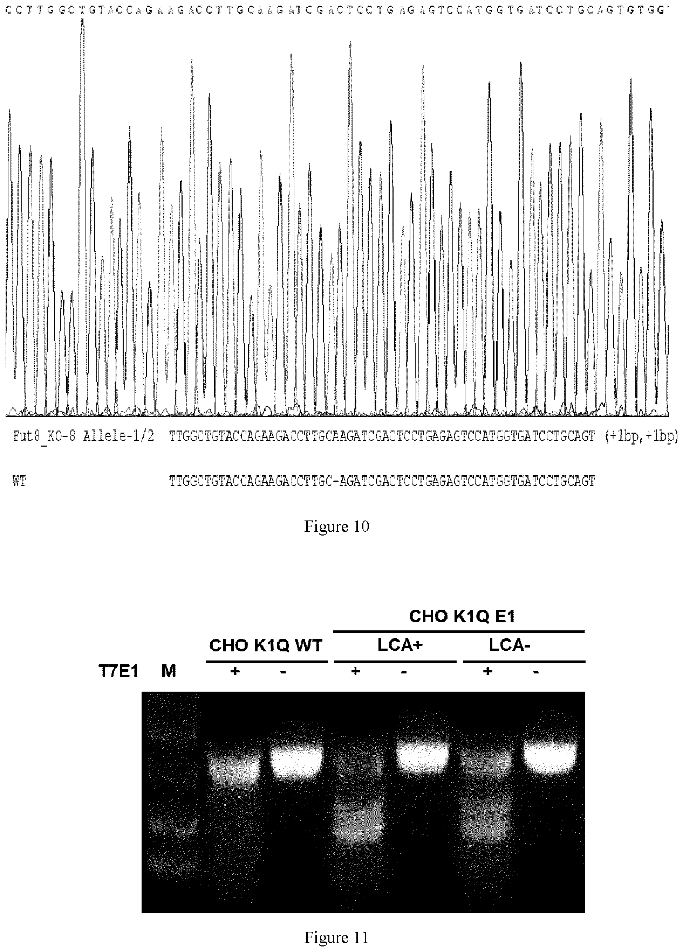

Further, the clonal sgcgFut8-2 gRNA/Cas9-electrotransfected cells, subjected to LCA pressure selection, were verified by FITC-LCA cell binding assay (Figure 9) and Sanger sequencing of the sgcgFut8-2-targeted Fut8 region (Figure 10), to obtain the CHOZN GS-/- cell line with homozygous knockout of Fut8 (Fut8_KO -8#).

### 2.3 Establishment of afucosylated CHO K1Q stable cell line

The afucosylated CHO K1Q cell line was established using the same method as used for the establishment of the afucosylated CHOZN GS-/- cell line. A total of three independent experiments were performed.

The *Fut8* gene in CHO K1Q cells (purchased from Kangsheng, Zhongshan) was successfully edited by electrotransfecting sgcgFut8-2 gRNA/Cas9 RNP, as verified by T7E1. As shown in Figure 11, the target DNA fragment was cleaved into two smaller fragments by the T7E1 enzyme. Figure 11 also showed that the efficiency of gene editing was further improved after LCA pressure selection, as the content of target DNA fragments was further reduced after cleavage by T7E1 enzyme.

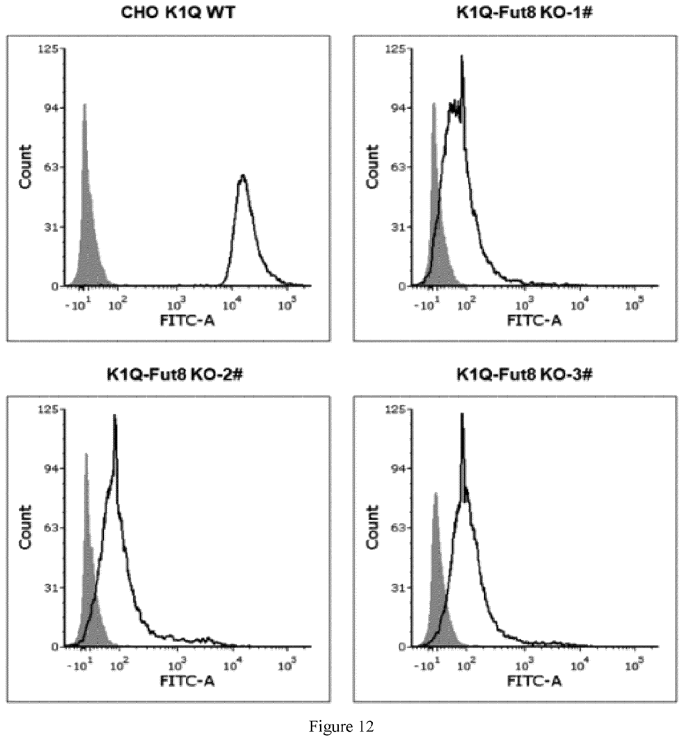

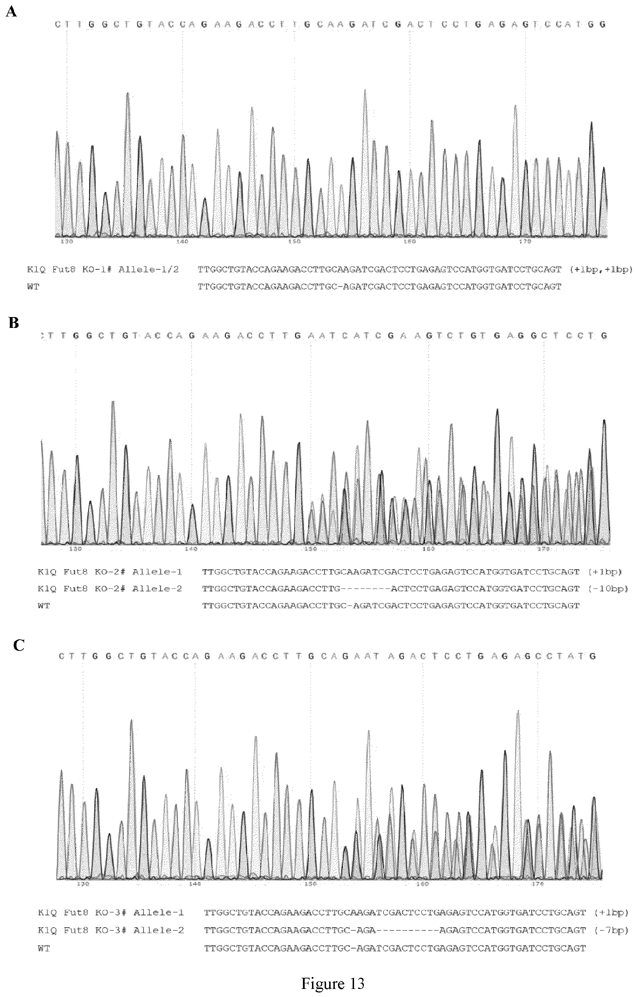

The clonal sgcgFut8-2 gRNA/Cas9-electrotransfected cells, subjected to LCA pressure selection, were verified by a FITC-LCA cell binding assay. As shown in Figure 12, the ability of CHO K1Q-Fut8 KO-1#, 2#, and 3# obtained from three independent experiments to bind FITC-LCA was significantly reduced, indicating that the cells had a reduced ability to produce fucosylated glycans or proteins. In addition, the *Fut8* region targeted by sgcgFut8-2 was subjected to Sanger sequencing. As shown in Figure 13, the sequencing results further confirmed that CHO K1Q-Fut8 KO-1#, 2#, and 3# were the CHO K1Q cell lines with homozygous knockout of Fut8.

### Example 3. Enhancement of ADCC and ADCP Activities of Neutralizing Antibody

This example related to the modification of anti-hepatitis B virus neutralizing antibody, specifically the Fc region, to increase the ADCC and ADCP activities thereof.

### 3.1 Preparation of A14 Fc variant

The inventor introduced an amino acid mutation into the heavy chain Fc region of the expressing A14 antibody using genetic engineering techniques, and obtained a series of A14 Fc variants; the inventor constructed these A14 Fc variants into the plasmid pCAGGS vector for eukaryotic expression (Addgene) and expressed them in the FCC#-293F cells constructed in Example 1 (with the knockout of *Fut8* gene), and obtained various afucosylated antibodies. In total, 33 different A14 Fc variants were constructed.

First, the absorbance of the protein at 280 nm was measured using Nanodrop one. The expression level of each A14 antibody variant was evaluated based on the amount of antibody per ml of cell culture medium, and the results were shown in Table 6. Each "+" in Table 6 represented 10 µg/ml. Based on the results in Table 6, the A14 antibody variants with higher expression levels were selected for subsequent experiments.

**Table 6. Constructed A14 Variants and Expression Levels thereof**

| **ID** | **A14 Fc Variant** | **Type of mutation** | **Expression Level** |
|---|---|---|---|
| 1 | DANA | D265A, N297A | + |
| 2 | WT | wild type with no mutation | +++++ |
| 3 | GA | G236A | ++++ |
| 4 | SD | S239D | +++++ |
| 5 | IE | I332E | +++++ |
| 6 | GASD | G236A, S239D | ++++ |
| 7 | GAIE | G236A, I332E | ++++ |
| 8 | SQIE | S239Q, I332E | +++ |
| 9 | GASQIE | G236A, S239Q, I332E | +++ |
| 10 | SQALIE | S239Q, A330L, I332E | +++ |
| 12 | FLRPLVYLPL | F243L, R292P, L235V, Y300L, P396L | +++ |
| 13 | FLRPYLVIPL | F243L, R292P, Y300L, V305I, P396L | +++ |
| 14 | LS | M428L, N434S | +++++ |
| 15 | YTE | M252Y, S254T, T256E | +++++ |
| 16 | GAALIE | G236A, A330L, I332E | +++ |
| 17 | GASQALIE | G236A, S239Q, A330L, I332E | ++++ |
| 18 | A14 GALS | G236A, M428L, N434S | +++++ |
| 19 | A14 GA YTE | G236A, M252Y, S254T, T256E | +++++ |
| 20 | SEIE | S239E, I332E | - |
| 21 | GASDIE | G236A, S239D, I332E | - |
| 22 | SDIE | S239D, I332E | - |
| 23 | SDALIE | S239D, A330L, I332E | - |
| 24 | GASDALIE | G236A, A330L, S239D, I332E | - |
| 25 | afuco-A14 WT | Afucosylated modification | ++++ |
| 26 | afuco-A14 GA | Afucosylated modification, G236A | ++++ |
| 27 | afuco-A14 SD | Afucosylated modification, S239D | - |
| 28 | afuco-A14 GAIE | Afucosylated modification, G236A, I332E | ++ |
| 29 | afuco-A14 GAALIE | Afucosylated modification, G236A, A330L, I332E | +++ |
| 30 | afuco-A14 LS | Afucosylated modification, M428L, N434S | +++++ |
| 31 | afuco-A14 YTE | Afucosylated modification, M252Y, S254T, T256E | +++++ |
| 32 | afuco-A14 GA LS | Afucosylated modification, G236A, M428L, N434S | +++++ |
| 33 | afuco-A14 GA YTE | Afucosylated modification, G236A, M252Y, S254T, T256E | +++++ |

### 3.2 Evaluation of ADCC and ADCP activities of A14 Fc variants

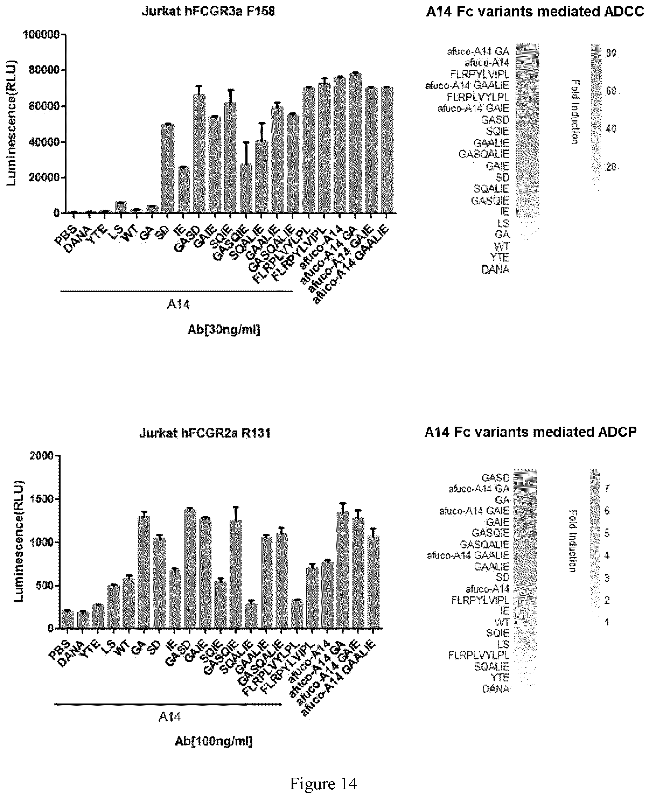

The ADCC and ADCP activities of the A14 Fc variants were evaluated using the transgenic cell lines of Jurkat/NFAT-luc2p/FcγRIIIa F158 and Jurkat/NFAT-luc2p/FcγRIIa R131 expressing the Fcy receptor as effector cells, which were conducted by the inventor based on a Jurkat parent cell (purchased from Cell Culture Center, Institute of Basic Medical Sciences, Chinese Academy of Medical Sciences), and the target cell was the CHO (CHO-59C) stably expressing the A14-binding epitope. An Fc variant antibody (DANA) that has lost the binding activity to the Fcy receptor was used as a control in the experiment. For the evaluation of ADCC and ADCP activities, the concentration of A14 Fc mutant antibody was respectively set at 30 ng/ml and 100 ng/ml, respectively, and the ratio of effector cells to target cells was 6:1. The relative luciferase activity (RLU) expressed by the cells was detected after incubation for 6 h at 37°C (Figure 14).

In the experiment, the luciferase activity value RLU induced by A14 DANA was set to 1, and the relative ADCC and ADCP activities induced by other A14 Fc mutations were compared.

As shown in Figure 14, afuco-A14 GA exhibited excellent performance in mediating both ADCC and ADCP activities. In mediating both ADCC and ADCP, afuco-A14 GA was not only better than either afuco-A14 with only afucosylated modification, or the Fc variant of amino acid mutation with only GA mutation, but it was also superior to antibody variants with other mutations based on afucosylated modification and GA mutation, such as afuco-A14 GAIE and afuco-A14 GAALIE. This result was unexpected. For example, as shown in Figure 14, the effect of GAIE was significantly better than GA in mediating ADCC, but upon further addition of the afucosylated modification, the effect of afuco-A14 GAIE was not as good as that of afuco-A14 GA.

### 3.3 Evaluation of dose-effect relationship of ADCC activity of A14 Fc variants

Based on the results of the preliminary evaluation in section 3.2, the A14 Fc variants afuco-A14, afuco-A14 GA, A14-GASD, A14-FLRPYLVIPL, A14-FLRPLVYLPL, and A14-GAIE, with relatively higher activities of ADCC and ADCP, were further evaluated in terms of dose-effect relationship of ADCC activity. An unmodified A14 antibody (A14 WT) was used as a control.

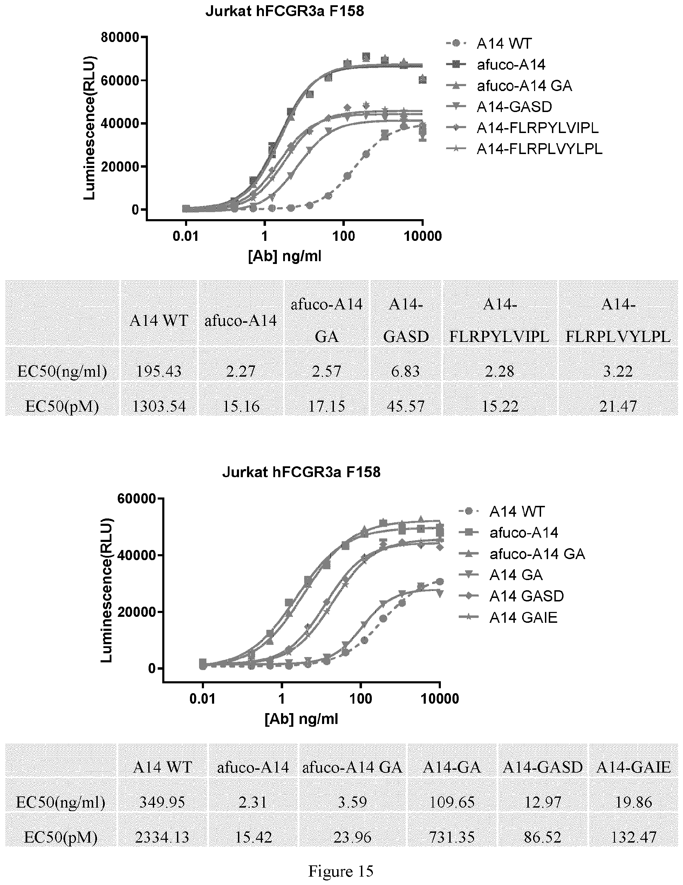

The initial protein amount of the antibody was 10 µg/ml, and 11 gradient dilution samples of different concentrations were prepared at a 3-fold dilution ratio. For the experiment, the Jurkat/NFAT-luc2p/FcγRIIIa F158 transgenic cell line (stably expressing NFAT-luc2p and FcγRIIIa F158 in Jurkat cells, wherein the Jurkat cells were purchased from Cell Bank, China Center for Type Culture Collection of the Chinese Academy of Sciences) was used as the effector cell, and the CHO cell stably expressing the A14 binding epitope (CHO-59C) was used as the target cell. The ratio of effector cells to target cells was 6:1. Negative control wells (i.e., wells containing only PBS without antibody) and background control wells were set up in the experiment. Using GraphPad Prism software, the curve was plotted using a four-parameter fitted regression model with the relative light units (RLU) on the y-axis and the log value of the antibody concentration (Log10) on the x-axis, and the EC50 value for the ADCC effect of the antibody was calculated. The candidate antibodies were evaluated and the results were shown in Figure 15, in which the afucosylated A14 antibody variants afuco-A14 and afuco-A14 GA exhibited the optimal ADCC activity.

### 3.4 Evaluation of ADCP activity of the A14 Fc variant

In the ADCP experiment, the afuco-A14, afuco-A14 GA, and A14-GASD mutant antibodies were further analyzed, and A14 WT was used as a control.

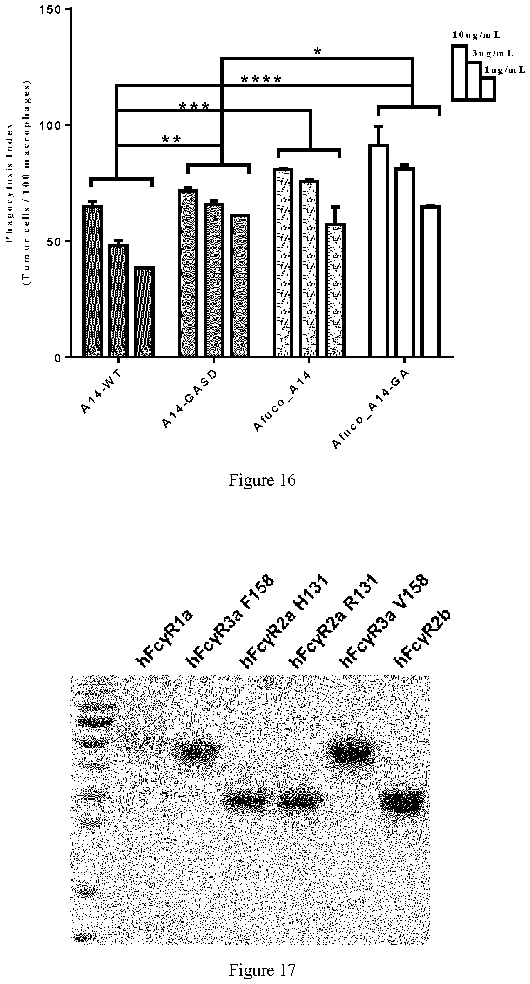

The effector cell used was a macrophage derived from a mouse bone marrow-derived macrophage induced to differentiate *in vitro;* the target cell was a 293F cell expressing the A14 binding epitope (293F-59C). The effector cell was labelled with an anti-mouse F4/80 monoclonal antibody with red fluorescent Alexa Fluor^{®} 647, and the arget cell was labelled with a green fluorescent dye CFSE. A negative control (PBS) was set up. The target cell was incubated with A14 Fc mutant antibody (10 µg/mL) and then added to the effector cell culture plates at 2 × 10⁵ cells/well for both effector and target cells, and the ratio of effector cells to target cells (E:T) was 1:1. After incubation for 2 h at 37°C, the culture supernatant was removed, and the target cells that were not phagocytosed by macrophages were washed away. Fluorescence images were taken using a NikonA1 confocal microscope, and the phagocytosis index, i.e. the number of positive target cells (green fluorescence) per 100 macrophages (red fluorescence), was counted. The results showed that afuco-A14 GA mediated the strongest phagocytosis of target cells 293F-59C by macrophages (ADCP) (Figure 16).

Combining the above results for the ADCC and ADCP assays with the expression levels of the antibodies, the afuco-A14-GA antibody was selected as a lead candidate for enhancing immune effect.

### Example 4. Affinity Determination of A14 Fc Mutant Antibodies for Human FcyRs Receptors 4.1 Recombinant expression of human FcyRs protein in vitro

To study the affinity between the A14 Fc variant and the FcyR receptor, the human FcγR receptor with a 6xHis tag at the C-terminus was recombinantly expressed in the 293F cell and purified by a Ni column.

### 4.2 Affinity determination of A14 Fc mutant antibody for FcyRs receptors

Biacore was used to analyse the affinity of several A14 Fc variants obtained from primary screening for multiple human FcyRs, including the activating receptors hFcγR1a, hFcyR2a H131, hFcyR2a R131, hFcyR3a V158, and hFcyR3a F158, and the inhibitory receptor hFcyR2b. V158 and R131 were high-affinity receptor SNPs, while F158 and R131 were low affinity receptor SNPs. The ADCC activity of the antibody variant was correlated with the affinity for hFcyR3a V158 and hFcyR3a F158 thereof; while the ADCP activity of the antibody variant was correlated with the affinity for hFcyR2a H131 and hFcyR2a R131 thereof. The results obtained were summarised in Table 7.

**Table 7 Biacore analysis of the affinity of the A14 Fc variant for human FevRs (KD M)**

| **Antibody ID** | **hFcγRIa** | **hFcγRIIa (H131)** | **hFcγRIIa (R131)** | **hFcγRIIb** | **hFcγRIIIa (V158)** | **hFcγRIIIa (F158)** |
|---|---|---|---|---|---|---|
| A14 WT | 5.35E-07 | 5.08E-07 | 7.13E-07 | 1.60E-06 | 6.72E-07 | 2.77E-06 |
| afuco-A14 WT | 5.52E-07 | 5.28E-07 | 4.73E-07 | 7.53E-07 | 5.57E-08 | 1.77E-07 |
| A14 GA | 5.36E-07 | 1.29E-07 | 1.87E-07 | 2.13E-06 | 1.28E-06 | 3.72E-06 |
| afuco-A14 GA | 7.18E-07 | 5.44E-08 | 1.39E-07 | 1.04E-06 | 6.67E-08 | 2.06E-07 |
| A14 GASD | 5.13E-07 | 1.44E-07 | 8.22E-08 | 8.29E-07 | 2.47E-07 | 6.85E-07 |
| A14 GAIE | 5.53E-07 | 1.18E-08 | 9.15E-08 | 7.26E-07 | 2.38E-07 | 5.13E-07 |

As shown in Table 7, afuco-A14 GA had enhanced affinity for the activating receptors hFcγR2a H131, hFcγR2a R131, hFcyR3a V158, and hFcγR3a F158 compared to wild type A14 (A14 WT).

Combining the above results, afuco-A14 GA was selected as the lead candidate molecule for enhancing immune effect. On this basis, further optimization was performed to improve the half-life of the antibody.

### Example 5. Genetic Engineering Modification to Obtain A14 Fc Variant with Prolonged Half-life

Based on the afuco-A14 GA antibody obtained according to the evaluation of previous studies, further genetic engineering modification was conducted to obtain the afuco-A14 GA LS and afuco-A14 GA YTE variants to prolong the half-life of the antibody *in vivo.*

Using the Jurkat cell-based luciferase reporter system for ADCC and ADCP, it was investigated whether the ADCC and ADCP activity of the antibody was affected by the introduction of the new mutations shown above.

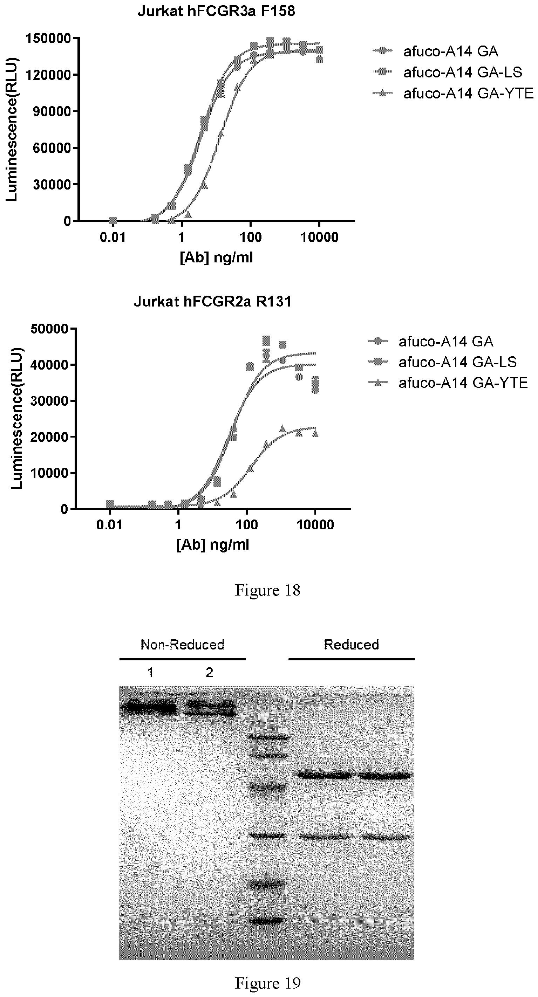

The results were shown in Figure 18. Compared to A14 WT and the precursor antibody afuco-A14 GA, the introduction of the YTE mutation in the Fc fragment of the antibody significantly reduced both the ADCC and ADCP activities of the antibody, affecting the immune effector activity of the precursor antibody. In contrast, the introduction of the LS mutation did not significantly alter ADCC and ADCP activities. These results indicated that afuco-A14 GA LS was the optimal candidate molecule for immune effector enhancement and half-life prolongation.

### Example 6. Physicochemical Properties, Biological Activity, and In Vivo Half-Life Studies of afuco-A14 GA LS Molecule

In this example, various properties and functions of the afuco-A14 GA LS antibody were further tested and compared to the unmodified A14 antibody.

### 6.1 SDS-PAGE analysis of expressed A14-WT and afuco-A14 GA LS

The A14 WT and afuco-A14 GA LS antibodies were expressed in 293F and FCC# cells, respectively, and the proteins were purified with protein A and quantified. Then, 2 µg of each sample was taken for SDS-PAGE electrophoresis analysis, respectively. The results were shown in Figure 9.

### 6.2 Analysis of affinity of afuco-A14 GA LS for human FcyRs

The affinities of afuco-A14 GA LS for hFcγRIa, hFcγRIIa H131, hFcγRIIa R131, hFcγRIIb, hFcγRIIIa V158, and hFcγRIIIa F158 were analysed using Biacore, according to the method described in Example 4. Specifically, Protein G was coupled on a CM5 chip via amine, and then the A14 WT antibody and the afuco-A14 GA LS antibody were captured, respectively. Different concentrations of recombinant hFcγR were used as the mobile phase over the flow path to allow binding and dissociation with the antibodies on the chip at pH 7.0. The affinity was then calculated by fitting the data using a 1:1 Langmuir binding model. The results were shown in Table 8.

As shown in Table 8, the affinity of afuco-A14 GA LS for the activating receptors hFcγRIIa and hFcγRIIIa was significantly enhanced compared to A14 WT.

**Table 8. Analysis of affinity of afuco-A14 GA LS for human FcγRs (KD, M)**

| **Antibody ID** | **hFcγR1a** | **hFcγRIIa H131** | **hFcγRIIa R131** | **hFcγRIIb** | **hFcγRIIIa V158** | **hFcγRIIIa F158** |
|---|---|---|---|---|---|---|
| **A14 WT** | 7.11E-08 | 7.82E-07 | 7.82E-07 | 5.67E-06 | 9.71E-07 | 6.48E-06 |
| **afuco-A14 GA LS** | 1.73E-07 | 1.69E-07 | 2.04E-07 | 7.47E-06 | 1.23E-07 | 5.88E-07 |
| **Fold** | 0.41 | 4.63 | 3.83 | 0.76 | 7.89 | 11.02 |

### 6.3 Evaluation of ADCC and ADCP activities of afuco-A14 GA LS

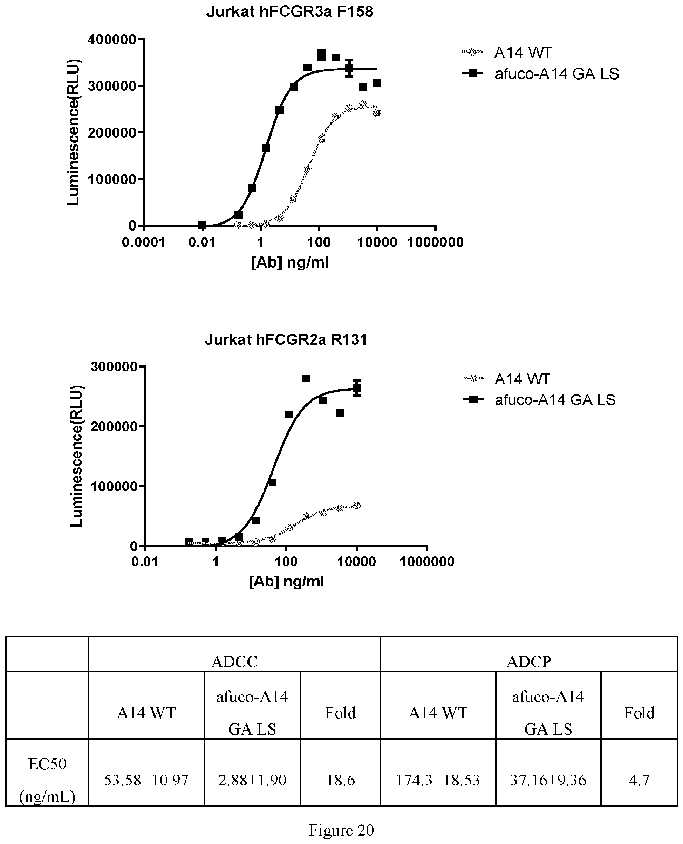

The same transgenic cell lines of Jurkat/NFAT-luc2p/FcγRIIIa F158 and Jurkat/NFAT-luc2p/FcyRIIa R131 as that in section 3.2 were used as effector cells, and a CHO cell stably expressing the A14 binding epitope (CHO-59C) was used as a target cell. The ratio of effector cells to target cells was 6:1. The initial concentration of the antibody was 10 mg/ml, and a total of 11 gradient dilution samples of different concentrations were prepared at a 3-fold dilution ratio for the comparison of the ADCC and ADCP activities between afuco-A14 GA LS and A14 WT antibodies. Negative control wells (wells containing only PBS without antibody) and background control wells were set up in the experiment. Using GraphPad Prism software, the curve was plotted using a four-parameter fitted regression model with the relative light units (RLU) on the y-axis and the log value of the antibody concentration (Log10) on the x-axis, and the EC50 value for the ADCC effect of the antibody was calculated. The results were shown in Figure 20.

The results showed that afuco-A14 GA LS significantly enhanced both ADCC and ADCP activities compared to A14, with at least 18.6-fold and 4.7-fold enhancement, respectively.

### 6.4 Evaluation of the binding activity of afuco-A14 GA LS to human FcRn

The half-life of the antibody *in vivo* is mainly determined by the affinity of the antibody Fc for FcRn on the surface of human endothelial cells. Binding of FcRn to the antibody IgG is pH dependent, binding only at about weakly acidic pH 6.0 and no binding at neutral pH. Therefore, the binding activity of afuco-A14 GA LS to human FcRn under acidic conditions was analysed by Biacore.

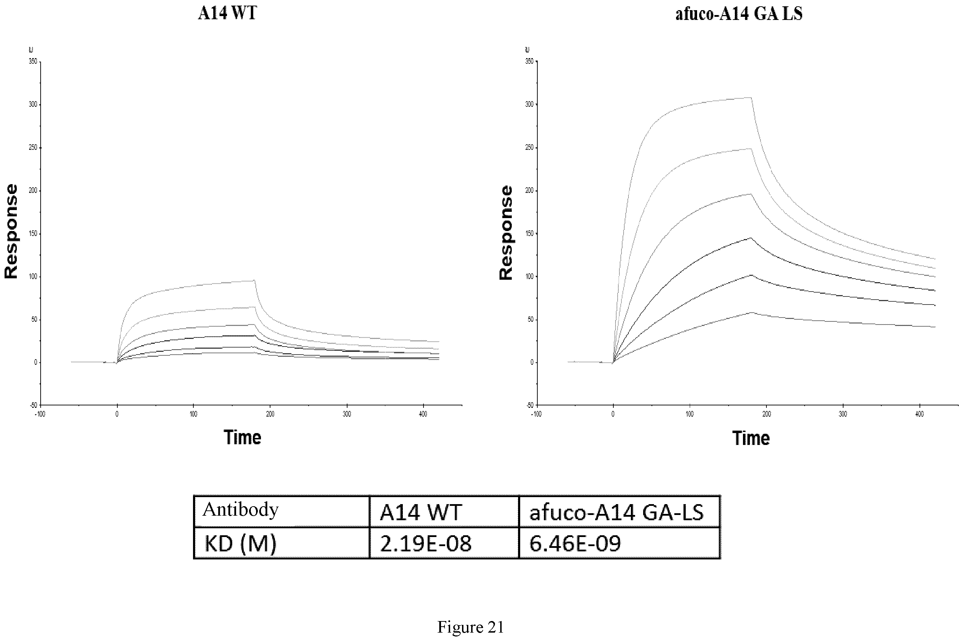

Specifically, recombinantly expressed hFcRn was coupled on a CM5 chip via amine, and then different concentrations of A14 WT antibody and the afuco-A14 GA LS antibody were used as the mobile phase over the flow path to allow binding and dissociation with the hFcRn on the chip at pH 6.0. The affinity was then calculated by fitting the data using a 1:1 Langmuir binding model. The results were shown in Figure 21.

As shown in Figure 21, it was observed that afuco-A14 GA LS exhibited a stronger binding affinity for hFcRn under acidic conditions compared to A14 WT, with a 3.4-fold increase in the KD value.

### 6.5 Analysis of affinity of afuco-A14 GA LS antibody for target antigen

Since the sequence modification of afuco-A14 GA LS was limited to the Fc region and had the same Fab for recognising the HBV Pre-S1 epitope as A14 WT, the binding activity to the target antigen Pre-S1 should theoretically be the same for both antibodies. However, the ability of afuco-A14 GA LS to bind to the target antigen was confirmed experimentally.

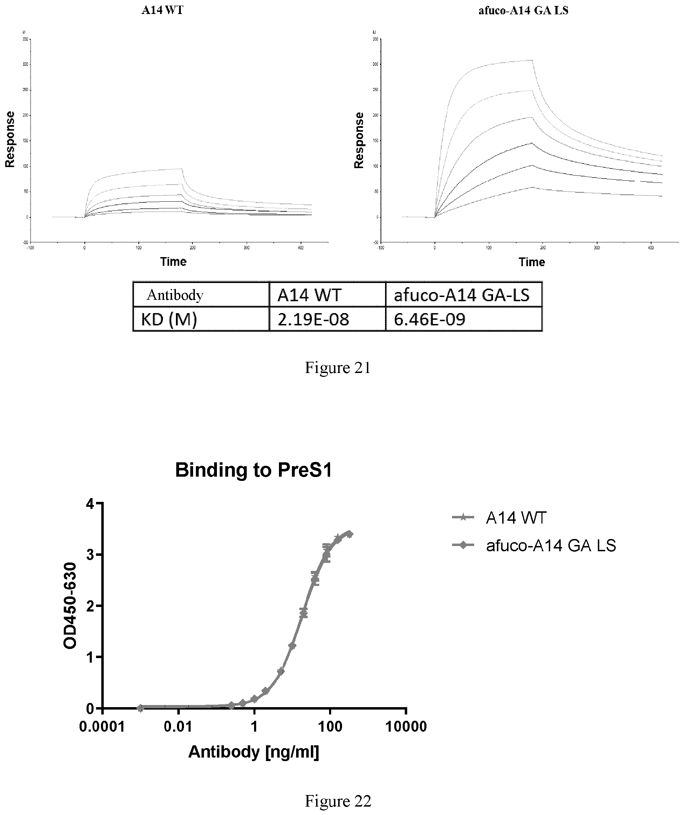

The relative binding activity of the afuco-A14 GA LS to the antigen peptide NC36b (pre-S1) was determined using an ELISA binding activity assay, and the results were shown in Figure 22. The antigen peptide was synthesised by Beijing Zhongke Yaguang Biotechnology Co. Ltd, and the sequence was as described in paragraph 019 of WO2016188386A1. The results in Figure 22 showed that the binding activity of afuco-A14 GA LS to Pre-S1 was comparable to that of A14 WT and was not significantly changed.

### 6.6 In vitro evaluation of antiviral activity

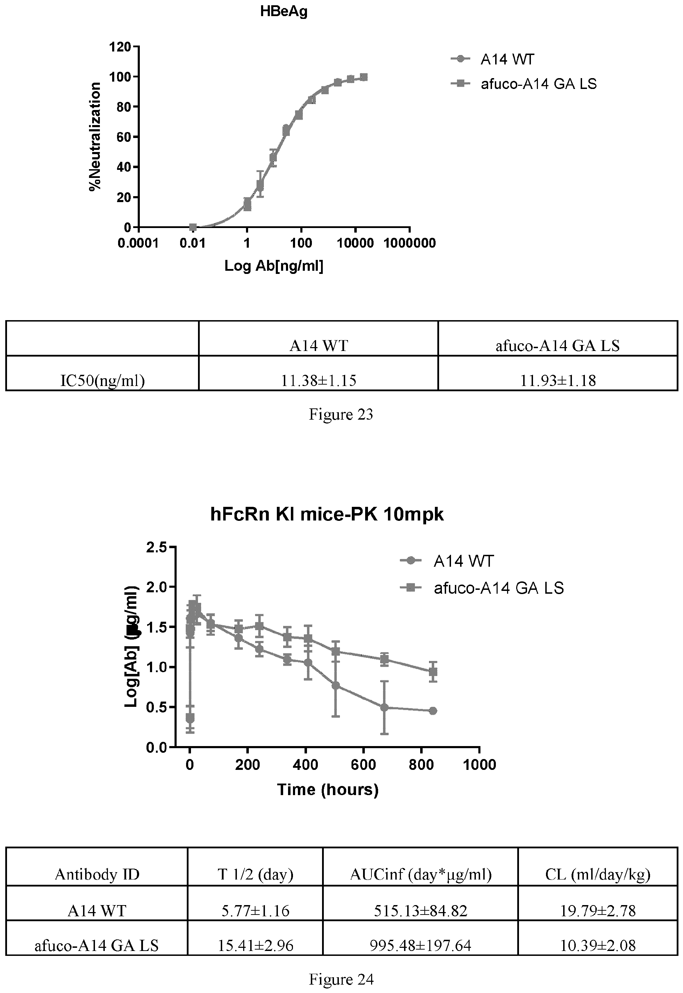

An HBV infection system using human HepG2-hNTCP cells prepared by the inventor as a host cell was used. Serial dilutions of afuco-A14 GA LS and A14 WT were mixed with equal volumes of recombinant HBV D genotype virus (prepared as described in Yan, H., et al., Sodium taurocholate cotransporting polypeptide is a functional receptor for human hepatitis B and D virus. Elife, 2012. 1: p. e00049) and used to infect HepG2-hNTCP cells. The cell culture supernatant was collected on day 5 post-infection. The HBeAg secretion in the cell culture supernatant was detected using an HBeAg antigen ELISA kit (Beijing Wantai Biological Pharmacy Co., Ltd.), and the percentage of inhibition at different antibody concentrations was calculated using the HBeAg secretion level of the virus infection control group (containing only PBS without added antibody) as the base. Using GraphPad Prism software, the curve was plotted using a four-parameter fitted regression model with the log value of the concentration on the x-axis and the percentage of inhibition on the y-axis, and the IC50 value was calculated. The results showed that afuco-A14 GA LS and A14 WT had comparable IC50 values of approximately 11.0 ng/ml (Figure 23).

### 6.7 In vivo half-life study of afuco-A14 GA LS in hFcRn KI mice

The half-life of afuco-A14 GA LS was further investigated in mice. Human FcRn knock-in (hFcRn KI) mice expressing human FcRn were used in this experiment. The experiment was set up with A14 WT and afuco-A14 GA LS groups, with 6 mice per group (half male and half female). The antibody dose was 10 mg/kg and was administered by intraperitoneal injection.

Blood samples were collected from mice at the following time points: pre-dose, and post-dose at 10min, 1h, 3h, 10h, 1d, 3d, 7d, 10d, 14d, 17d, 21d, 28d, and 35d. The drug concentration in the serum of mice was measured using an ELISA method. The results were shown in Figure 24. For A14 WT, the T_{1/2} was 5.77 ± 1.16 days, the AUC_{Inf} was 515.13 ± 84.82 days*µg/mL, and the terminal clearance rate (CL) was 19.79 ± 2.78 mL/day/kg; while for afuco-A14 GA LS, the T_{1/2} was 15.41 ± 2.96 days, the AUC_{Inf} was 995.48 ± 197.64 days*µg/mL, and the CL was 10.39 ± 2.08 mL/day/kg. As seen from the results, the half-life of afuco-A14 GA LS was prolonged by about 2.67-fold as compared to that of A14 WT.

### 6.8 Evaluation of antiviral activity of afuco-A14 GA LS in mice

To investigate the therapeutic efficacy of afuco-A14 GA LS against established HBV infection, the efficacy of afuco-A14 GA LS administered alone and in combination with entecavir, was evaluated in a liver-humanized chimeric hFRG (Fah^{-/-} Rag2^{-/-} /IL2rg^{-/-}) mouse model. Entecavir is a hepatitis B virus reverse transcriptase inhibitor that treats hepatitis B by inhibiting viral replication. The disadvantage of entecavir is the rebound of viral replication upon discontinuation of treatment.

A control group (saline), an afuco-A14 GA LS monotherapy group (10 mg/kg), an entecavir (ETV) monotherapy group (ETV, 0.1 mg/kg), and a combination therapy group of afuco-A14 GA LS (10 mg/kg) and entecavir (0.1 mg/kg) were set up in the experiment, respectively. Mice were administered (afuco-A14 GALS was administered subcutaneously and ETV was administered orally) starting on day 28 (D28) post HBV infection (HBV D genotype, 2 × 10⁹ GE/mouse). Thereafter, afuco-A14 GA LS was administered subcutaneously once a week (D35, D42, D49, D56, D63, and D70), while ETV was administered once a day until D70. The observation period continued after discontinuation until D91.

Plasma from experimental mice was collected by venous blood sampling, and then qPCR was used to quantitatively detect the level of HBV DNA in the plasma of the mice. The experimental results showed that the HBV DNA levels in the serum of all mice in the administered group were significantly reduced compared to the control group (saline). Specifically, at the final dose (D70), the viral DNA levels decreased approximately 7.3-fold and 436-fold in the afuco-A14 GA LS monotherapy group and the ETV monotherapy group, respectively. The combination of afuco-A14 GA LS and ETV further reduced the levels of serum viral titres by 855-fold. Viral titres rebounded after discontinuation in all treatment groups, but remained below baseline of viral titres at D28. The mean viral titre values for each group at the end of the study (D91, 21 days post-discontinuation) were reduced by the following factors compared to the starting point of treatment (D28): 2.43-fold in the afuco-A14 GA LS monotherapy group, 7.15-fold in the ETV monotherapy group, and 12.6-fold in the combination therapy group. These results indicated that the antibody variant of the present invention was more effective in combination with ETV than either of them alone, and that this advantage in antiviral efficacy persisted for up to 21 days post-discontinuation of treatment.

### Sequence Information

| SEQ ID NO: | Name | Description | Sequence |
|---|---|---|---|
| | Antibody sequence of afuco-A14 GA LS | | |
| 1 | VH aa | Amino acid sequence of | QVQLQQSGPGLVKPSQTLSLTCAISGDSVSSNSAAWNWIRQSPS RGLEWLGRTYYRSKWYNDYAVSVKSRITINPDTSKNQFSLQLN |
| | | heavy chain variable region | SVTPEDTAVYYCARGTRWGMDVWGQGTLVTVSS |
| 2 | CH aa | Amino acid sequence of heavy chain constant region (The mutations of G236A, M428L, and N434S are boxed.) | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPELLAGPSVFLFPPKPKDT LMISRTPEVTCVVVDVSHEDPEVKFNWYVDGVEVHNAKTKPRE EQYNSTYRVVSVLTVLHQDWLNGKEYKCKVSNKALPAPIEKTI SKAKGQPREPQVYTLPPSRDELTKNQVSLTCLVKGFYPSDIAVE WESNGQPENNYKTTPPVLDSDGSFFLYSKLTVDKSRWQQGNVF SCSVLHEALHSHYTQKSLSLSPGK |
| 3 | VH DNA | DNA sequence of heavy chain variable region | CAGGTACAGCTGCAGCAGTCAGGTCCAGGACTGGTGAAGCC CTCGCAGACCCTCTCACTCACCTGTGCCATCTCCGGGGACAG TGTCTCTAGCAACAGTGCTGCTTGGAACTGGATCAGGCAGTC CCCATCGAGAGGCCTTGAGTGGCTGGGAAGGACATACTACA GGTCCAAGTGGTATAATGATTATGCAGTATCTGTGAAAAGTC GAATAACCATCAACCCAGACACATCCAAGAACCAGTTCTCCC TGCAGCTGAACTCTGTGACTCCCGAGGACACGGCTGTGTATT ACTGTGCAAGAGGAACACGTTGGGGTATGGACGTCTGGGGC CAAGGGACCCTGGTCACCGTCTCTTCA |
| 4 | CH DNA | DNA sequence of heavy chain constant region (The codons of mutations of G236A, M428L, and N434S are boxed.) | GCTAGCACCAAGGGCCCATCGGTCTTCCCCCTGGCACCCTCC TCCAAGAGCACCTCTGGGGGCACAGCGGCCCTGGGCTGCCTG GTCAAGGACTACTTCCCCGAACCGGTGACGGTGTCGTGGAAC TCAGGCGCCCTGACCAGCGGCGTGCACACCTTCCCGGCTGTC CTACAGTCCTCAGGACTCTACTCCCTCAGCAGCGTGGTGACC GTGCCCTCCAGCAGCTTGGGCACCCAGACCTACATCTGCAAC GTGAATCACAAGCCCAGCAACACCAAGGTGGACAAGAAGGT TGAGCCCAAATCTTGTGACAAAACTCACACATGCCCACCGTG CCCAGCACCTGAACTCCTGGCGGGACCGTCAGTCTTCCTCTT CCCCCCAAAACCCAAGGACACCCTCATGATCTCCCGGACCCC TGAGGTCACATGCGTGGTGGTGGACGTGAGCCACGAAGACC CTGAGGTCAAGTTCAACTGGTACGTGGACGGCGTGGAGGTGC ATAATGCCAAGACAAAGCCGCGGGAGGAGCAGTACAACAGC ACGTACCGTGTGGTCAGCGTCCTCACCGTCCTGCACCAGGAC TGGCTGAATGGCAAGGAGTACAAGTGCAAGGTCTCCAACAA AGCCCTCCCAGCCCCCATCGAGAAAACCATCTCCAAAGCCAA AGGGCAGCCCCGAGAACCACAGGTGTACACCCTGCCCCCATC CCGGGATGAGCTGACCAAGAACCAGGTCAGCCTGACCTGCCT GGTCAAAGGCTTCTATCCCAGCGACATCGCCGTGGAGTGGGA GAGCAATGGGCAGCCGGAGAACAACTACAAGACCACGCCTC CCGTGCTGGACTCCGACGGCTCCTTCTTCCTCTACAGCAAGCT CACCGTGGACAAGAGCAGGTGGCAGCAGGGGAACGTCTTCT CATGCTCCGTGCTGCATGAGGCTCTGCACAGCCACTACACGC AGAAGAGCCTCTCCCTGTCTCCGGGTAAATGA |
| 5 | VL aa | Amino acid sequence of light chain variable region | QSVLTQPPSASGTPGQRVTISCSGSSSNIGSYYVYWYQQLPGTAP KLLIYGNNQRPSGVPDRFSGSKSGTSASLAITGLQAEDEADYYC QSYDSSLSGVIFGGGTKLTVL |
| 6 | CL aa | Amino acid sequence of light chain constant region | GQPKAAPSVTLFPPSSEELQANKATLVCLISDFYPGAVTVAWKA DSSPVKAGVETTTPSKQSNNKYAASSYLSLTPEQWKSHRSYSCQ VTHEGSTVEKTVAPTECS |
| 7 | VL DNA | DNA sequence of | CAGAGCGTCCTGACCCAGCCACCCAGCGCAAGCGGCACCCC CGGCCAGCGAGTGACCATTTCATGCAGCGGCAGCAGCAGCA |
| | | light chain variable region | ACATTGGGTCCTACTATGTGTACTGGTATCAGCAGCTGCCCG GAACAGCTCCTAAGCTGCTGATCTACGGAAACAATCAGCGGC CAAGCGGCGTCCCCGACAGATTCTCTGGCAGTAAATCAGGGA CTAGCGCCTCCCTGGCTATCACCGGACTGCAGGCAGAGGACG AAGCCGATTACTATTGCCAGTCTTATGATAGCTCCCTGAGTG GGGTGATTTTTGGCGGGGGAACTAAACTGACCGTCCTG |
| 8 | CL DNA | DNA sequence of light chain constant region | GGTCAGCCCAAGGCTGCCCCCTCGGTCACTCTGTTCCCGCCC TCCTCTGAGGAGCTTCAAGCCAACAAGGCCACACTGGTGTGT CTCATAAGTGACTTCTACCCGGGAGCCGTGACAGTGGCCTGG AAGGCAGATTCCAGCCCCGTCAAGGCGGGAGTGGAGACCAC CACACCCTCCAAACAAAGCAACAACAAGTACGCGGCCAGCA GCTATCTGAGCCTGACGCCTGAGCAGTGGAAGTCCCACAGAA GCTACAGCTGCCAGGTCACGCATGAAGGGAGCACCGTGGAG AAGACAGTGGCCCCTACAGAATGTTCATGA |
| 9-30 | Reference to above | | |
| 31 | hIgG1 aa | Amino acid sequence of heavy chain constant region of human IgG1 (Normal font represents CH1, underlined represents hinge region, bold represents CH2, and italic represents CH3.) | |
| 32 | A14 HCDR1 | | SNSAAWN |
| 33 | A14 HCDR2 | | RTYYRSKWYNDYAVS |
| 34 | A14 HCDR3 | | GTRWGMDV |
| 35 | A14 LCDR1 | | SGSSSNIGSYYVYWY |
| 36 | A14 LCDR2 | | GNNQRPS |
| 37 | A14 LCDR3 | | QSYDSSLSGVI |
| 38 | A14 CH aa | Amino acid sequence of heavy chain constant region of parent antibody | |
| 39 | Reference to above | | |

## Claims

1. An antibody variant specifically binding to a Pre-S1 domain of hepatitis B virus (HBV), having an enhanced Fc-mediated effector function, such as ADCC and/or ADCP activity, compared to a parent antibody thereof.

2. The antibody variant according to claim 1, wherein the antibody variant and the parent antibody are IgG1 antibodies.

3. The antibody variant according to claim 1 or 2, wherein the antibody has, relative to the parent antibody thereof, a characteristic selected from the group consisting of:
(1) a reduced amount of sugar chain containing 1,6-fucose or an absence of sugar chain containing 1,6-fucose, and/or one or more amino acid modifications selected from the group consisting of: L235V, S239E/D/Q, F243L, R292P, Y300L, V305I, A330L, I332E, and P396L; and
(2) optionally, an amino acid mutation of G236A.

4. The antibody variant according to claim 3, having relative to the parent antibody a mutation selected from any of the following groups (a) to (f):
(a) having a reduced amount of sugar chain containing 1,6-fucose or an absence of sugar chain containing 1,6-fucose;
(b) having a reduced amount of sugar chain containing 1,6-fucose or an absence of sugar chain containing 1,6-fucose, and having an amino acid mutation of G236A (GA mutation);
(c) having amino acid mutations of G236A and S239D (GASD mutation);
(d) having mutations of F243L, R292P, L235V, Y300L, and P396L (FLRPLVYLPL mutation);
(e) having mutations of F243L, R292P, Y300L, V305I, and P396L (FLRPYLVIPL mutation); and
(f) having mutations of G236A and I332E (GAIE mutation).

5. The antibody variant according to claim 3 or 4, wherein a reduction or absence of the sugar chain comprising 1,6-fucose is achieved by knocking out the *Fut8* gene in a host cell used to produce the antibody variant.

6. The antibody variant according to any one of claims 1 to 5, having a prolonged half-life relative to the parent antibody.

7. The antibody variant according to claim 6, comprising relative to the parent antibody one or more amino acid mutations selected from the group consisting of: M252Y, S254T, T256E, M428L, and N434S.

8. The antibody variant according to claim 7, comprising relative to the parent antibody an amino acid mutation selected from the following group (g) or (h):
(g) amino acid mutations of M428L and N434S (LS mutation); and
(h) amino acid mutations of M252Y, S254T, and T256E (YTE mutation).

9. The antibody variant according to any one of claims 1 to 8, having relative to the parent antibody:
(1) a reduced amount of sugar chain containing 1,6-fucose or an absence of sugar chain containing 1,6-fucose, and/or one or more amino acid mutations selected from the group consisting of: L235V, S239E/D/Q, F243L, R292P, Y300L, V305I, A330L, I332E, and P396L;
(2) optionally, an amino acid mutation of G236A; and
(3) optionally, one or more amino acid mutations selected from the group consisting of: M252Y, S254T, T256E, M428L, and N434S.

10. The antibody variant according to any one of claims 1 to 9, wherein the parent antibody and the antibody variant have CDR sequences as shown in SEQ ID NOs: 32 to 37.

11. The antibody variant according to claim 16, wherein the parent antibody is an A14 antibody, having a heavy chain variable region sequence as shown in SEQ ID NO: 1, a heavy chain constant region sequence as shown in SEQ ID NO: 38, a light chain variable region sequence as shown in SEQ ID NO: 5, and a light chain constant region sequence as shown in SEQ ID NO: 6.

12. An antibody specifically binding to a Pre-S1 domain of hepatitis B virus (HBV), comprising:
(1) three heavy chain CDR sequences as shown in SEQ ID NOs: 32, 33, and 34, and three light chain CDR sequences as shown in SEQ ID NOs: 35, 36, and 37; and
(2) a heavy chain constant region comprising an amino acid sequence as shown in SEQ ID NO: 2.

13. An isolated nucleic acid molecule, comprising a nucleotide sequence encoding an antibody variant according to any one of claims 1 to 18 or an antibody according to claim 12, preferably, comprising a nucleotide sequence as shown in SEQ ID NO: 4 to encode a heavy chain constant region.

14. An expression vector, comprising the isolated nucleic acid molecule according to claim 13.

15. A host cell, comprising the isolated nucleic acid molecule according to claim 13 or the expression vector according to claim 14.

16. A mammalian cell, **characterised in that** FUT8 in the cell is inactivated and the isolated nucleic acid molecule according to claim 13 or the expression vector according to claim 14 is introduced into the cell.

17. The mammalian cell according to claim 16, being selected from the group consisting of: a 293F cell, a CHOK1 cell, a CHOZN GS-/- cell, and a CHO K1Q cell.

18. A method for preparing an antibody, comprising the following steps:
(1) culturing the mammalian cell according to any one of claims 16 and 17 in a medium to obtain a culture comprising the antibody; and
(2) recovering the antibody from the culture.

19. A method for preparing a mammalian cell with *Fut8* gene knockout, comprising introducing into the mammalian cell:
(a) a gRNA targeting *Fut8* and a coding sequence of a Cas9 nuclease, or
(b) a complex of a gRNA targeting *Fut8* and a Cas9 nuclease,
wherein (b) is preferably introduced.

20. The method according to claim 19, wherein the nucleotide sequence of the sgRNA is selected from a nucleotide sequence as shown in SEQ ID NO: 10, SEQ ID NO: 19, SEQ ID NO: 20, SEQ ID NO: 21 or SEQ ID NO: 39.
